# EUROPEAN PATENT APPLICATION

(11) **EP 1 568 383 A2**
(43) Date of publication of application: **31.08.2005**
(21) Application number: 04004478.6
(22) Date of filing: 27.02.2004
(51) Int. Cl.: A61K 48/00, A61K 31/713, C12N 15/11, C07H 21/00

(54) **Use of an oligonucleotide or its active derivative for the preparation of a pharmaceutical composition for inhibiting the formation of metastases in cancer treatment**

(71) Applicant: Antisense Pharma GmbH, 93053 Regensburg (DE)
(72) Inventor: Schlingensiepen, Karl-Hermann, Dr., 93093 Donaustauf (DE); Schlingensiepen, Reimar, Dr., 93051 Regensburg (DE); Jachimczak, Piotr, Dr., 97074 Würzburg (DE); Stauder, Gerhard, Dr., 82538 Geretsried (DE); Bischof, Astrid, Dr., 93049 Regensburg (DE); Hafner, Michael, Dr., 93059 Regensburg (DE); Egger, Tamara, Dr., 93059 Regensburg (DE)
(74) Representative: Schreiber, Christoph

(57) **Abstract**

The present invention is related to pharmaceutical compositions for the inhibition of metastases and treatment of cancer such as bladder carcinoma, colon cancer, endometrial cancer, hepatocellular carcinoma, leukemia, lymphoma, melanoma, non-small cell lung cancer (NSCLC), ovarian cancer, pancreatic cancer, prostate cancer, soft tissue cancer. Another aspect of this invention are new antisense oligonucleotides inhibiting the formation of human interleukin 10 and their synthesis.

SEQUENCE LISTING

<110> Antisense Pharma GmbH

<120> Pharmaceutical Composition

<130> 032744ep CS/BM

<140> 04004478.6
<141> 2004-02-27

<160> 72

<170> PatentIn Ver. 2.1

<210> 1
<211> 14
<212> DNA
<213> Artificial Sequence

<220>
<223> Description of Artificial Sequence: antisense
      oligonucleotide

<400> 1
cgatagtctt gcag                                                   14


<210> 2
<211> 14
<212> DNA
<213> Artificial Sequence

<220>
<223> Description of Artificial Sequence: antisense
      oligonucleotide

<400> 2
gtcgatagtc ttgc                                                   14


<210> 3
<211> 14
<212> DNA
<213> Artificial Sequence

<220>
<223> Description of Artificial Sequence: antisense
      oligonucleotide

<400> 3
cttggacagg atct                                                   14


<210> 4
<211> 14
<212> DNA
<213> Artificial Sequence

<220>
<223> Description of Artificial Sequence: antisense
      oligonucleotide

<400> 4
ccaggaattg ttgc                                                    14

<210> 5
<211> 14
<212> DNA
<213> Artificial Sequence

<220>
<223> Description of Artificial Sequence: antisense
      oligonucleotide

<400> 5
cctcaatttc ccct                                                    14

<210> 6
<211> 14
<212> DNA
<213> Artificial Sequence

<220>
<223> Description of Artificial Sequence: antisense
      oligonucleotide

<400> 6
gatgtccact tgca                                                    14

<210> 7
<211> 14
<212> DNA
<213> Artificial Sequence

<220>
<223> Description of Artificial Sequence: antisense
      oligonucleotide

<400> 7
ctccaaatgt aggg                                                    14

<210> 8
<211> 14
<212> DNA
<213> Artificial Sequence

<220>
<223> Description of Artificial Sequence: antisense
      oligonucleotide

<400> 8
accttgctgt actg                                                    14

<210> 9
<211> 14
<212> DNA
<213> Artificial Sequence

<220>
<223> Description of Artificial Sequence: antisense
      oligonucleotide

<400> 9
gtagtacacg atgg                                                    14


<210> 10
<211> 14
<212> DNA
<213> Artificial Sequence

<220>
<223> Description of Artificial Sequence: antisense
      oligonucleotide

<400> 10
cacgtagtac acga                                                    14


<210> 11
<211> 14
<212> DNA
<213> Artificial Sequence

<220>
<223> Description of Artificial Sequence: antisense
      oligonucleotide

<400> 11
catgttggac agct                                                    14


<210> 12
<211> 14
<212> DNA
<213> Artificial Sequence

<220>
<223> Description of Artificial Sequence: antisense
      oligonucleotide

<400> 12
gcacgatcat gttg                                                    14


<210> 13
<211> 16
<212> DNA
<213> Artificial Sequence

<220>
<223> Description of Artificial Sequence: antisense
      oligonucleotide

<400> 13
tgtactctgc ttgaac                                                  16


<210> 14
<211> 18

<212> DNA
<213> Artificial Sequence

<220>
<223> Description of Artificial Sequence: antisense
      oligonucleotide

<400> 14
ctgatgtgtt gaagaaca                                                    18


<210> 15
<211> 16
<212> DNA
<213> Artificial Sequence

<220>
<223> Description of Artificial Sequence: antisense
      oligonucleotide

<400> 15
ctctgatgtg ttgaag                                                      16


<210> 16
<211> 16
<212> DNA
<213> Artificial Sequence

<220>
<223> Description of Artificial Sequence: antisense
      oligonucleotide

<400> 16
ggaagtcaat gtacag                                                      16


<210> 17
<211> 18
<212> DNA
<213> Artificial Sequence

<220>
<223> Description of Artificial Sequence: antisense
      oligonucleotide

<400> 17
catgtcgata gtcttgca                                                    18


<210> 18
<211> 18
<212> DNA
<213> Artificial Sequence

<220>
<223> Description of Artificial Sequence: antisense
      oligonucleotide

<400> 18
agctgaagca atagttgg                                                    18

<210> 19
<211> 18
<212> DNA
<213> Artificial Sequence

<220>
<223> Description of Artificial Sequence: antisense
      oligonucleotide

<400> 19
gtcatagatt tcgttgtg                                                    18


<210> 20
<211> 18
<212> DNA
<213> Artificial Sequence

<220>
<223> Description of Artificial Sequence: antisense
      oligonucleotide

<400> 20
ctccactttt aacttgag                                                    18


<210> 21
<211> 18
<212> DNA
<213> Artificial Sequence

<220>
<223> Description of Artificial Sequence: antisense
      oligonucleotide

<400> 21
tgctgtattt ctggtaca                                                    18


<210> 22
<211> 14
<212> DNA
<213> Artificial Sequence

<220>
<223> Description of Artificial Sequence: antisense
      oligonucleotide

<400> 22
cacacagtag tgca                                                        14


<210> 23
<211> 14
<212> DNA
<213> Artificial Sequence

<220>
<223> Description of Artificial Sequence: antisense
      oligonucleotide

<400> 23
gcacacagta gtgc                                                    14


<210> 24
<211> 17
<212> DNA
<213> Artificial Sequence

<220>
<223> Description of Artificial Sequence: antisense
      oligonucleotide

<400> 24
gcttgctcag gatctgc                                                 17


<210> 25
<211> 14
<212> DNA
<213> Artificial Sequence

<220>
<223> Description of Artificial Sequence: antisense
      oligonucleotide

<400> 25
tactcttcgt cgct                                                    14


<210> 26
<211> 14
<212> DNA
<213> Artificial Sequence

<220>
<223> Description of Artificial Sequence: antisense
      oligonucleotide

<400> 26
cttggcgtag tact                                                    14


<210> 27
<211> 14
<212> DNA
<213> Artificial Sequence

<220>
<223> Description of Artificial Sequence: antisense
      oligonucleotide

<400> 27
gtaaacctcc ttgg                                                    14


<210> 28
<211> 19
<212> DNA
<213> Artificial Sequence

<220>

<223> Description of Artificial Sequence: antisense
      oligonucleotide

<400> 28
   gtctattttg taaacctcc                                                    19


<210> 29
<211> 20
<212> DNA
<213> Artificial Sequence

<220>
<223> Description of Artificial Sequence: antisense
      oligonucleotide

<400> 29
gcatgtctat tttgtaaacc                                                  20


<210> 30
<211> 18
<212> DNA
<213> Artificial Sequence

<220>
<223> Description of Artificial Sequence: antisense
      oligonucleotide

<400> 30
cggcatgtct attttgta                                                    18


<210> 31
<211> 14
<212> DNA
<213> Artificial Sequence

<220>
<223> Description of Artificial Sequence: antisense
      oligonucleotide

<400> 31
ggcatcaagg tacc                                                        14


<210> 32
<211> 14
<212> DNA
<213> Artificial Sequence

<220>
<223> Description of Artificial Sequence: antisense
      oligonucleotide

<400> 32
ctgtagaaag tggg                                                        14


<210> 33
<211> 18
<212> DNA

<213> Artificial Sequence

<220>
<223> Description of Artificial Sequence: antisense
      oligonucleotide

<400> 33
acaattctga agtagggt                                                    18


<210> 34
<211> 18
<212> DNA
<213> Artificial Sequence

<220>
<223> Description of Artificial Sequence: antisense
      oligonucleotide

<400> 34
tcaccaaatt ggaagcat                                                    18


<210> 35
<211> 20
<212> DNA
<213> Artificial Sequence

<220>
<223> Description of Artificial Sequence: antisense
      oligonucleotide

<400> 35
gctttcacca aattggaagc                                                  20


<210> 36
<211> 14
<212> DNA
<213> Artificial Sequence

<220>
<223> Description of Artificial Sequence: antisense
      oligonucleotide

<400> 36
ctggcttttg ggtt                                                        14


<210> 37
<211> 18
<212> DNA
<213> Artificial Sequence

<220>
<223> Description of Artificial Sequence: antisense
      oligonucleotide

<400> 37
tctgatatag ctcaatcc                                                    18

<210> 38
<211> 18
<212> DNA
<213> Artificial Sequence

<220>
<223> Description of Artificial Sequence: antisense
      oligonucleotide

<400> 38
tcctagtgga ctttatag                                                    18


<210> 39
<211> 16
<212> DNA
<213> Artificial Sequence

<220>
<223> Description of Artificial Sequence: antisense
      oligonucleotide

<400> 39
tttttcctag tggact                                                      16


<210> 40
<211> 19
<212> DNA
<213> Artificial Sequence

<220>
<223> Description of Artificial Sequence: antisense
      oligonucleotide

<400> 40
caattatcct gcacatttc                                                   19


<210> 41
<211> 16
<212> DNA
<213> Artificial Sequence

<220>
<223> Description of Artificial Sequence: antisense
      oligonucleotide

<400> 41
gcaattatcc tgcaca                                                      16


<210> 42
<211> 16
<212> DNA
<213> Artificial Sequence

<220>
<223> Description of Artificial Sequence: antisense
      oligonucleotide

<400> 42

gcagcaatta tcctgc                                                    16

<210> 43
<211> 14
<212> DNA
<213> Artificial Sequence

<220>
<223> Description of Artificial Sequence: antisense
      oligonucleotide

<400> 43
tggcattgta ccct                                                      14

<210> 44
<211> 14
<212> DNA
<213> Artificial Sequence

<220>
<223> Description of Artificial Sequence: antisense
      oligonucleotide

<400> 44
tgtgctgagt gtct                                                      14

<210> 45
<211> 16
<212> DNA
<213> Artificial Sequence

<220>
<223> Description of Artificial Sequence: antisense
      oligonucleotide

<400> 45
cctgctgtgc tgagtg                                                    16

<210> 46
<212> DNA
<213> Artificial Sequence

<220>
<223> Description of Artificial Sequence: antisense
      oligonucleotide

<400> 46

<210> 47
<211> 18
<212> DNA
<213> Artificial Sequence

<220>
<223> Description of Artificial Sequence: antisense
      oligonucleotide

<400> 47
tttagctgca tttgcaag                                                    18

<210> 48
<211> 14
<212> DNA
<213> Artificial Sequence

<220>
<223> Description of Artificial Sequence: antisense
      oligonucleotide

<400> 48
gccacttttc caag                                                        14

<210> 49
<211> 18
<212> DNA
<213> Artificial Sequence

<220>
<223> Description of Artificial Sequence: antisense
      oligonucleotide

<400> 49
cttcttttgc aagtctgt                                                    18

<210> 50
<211> 18
<212> DNA
<213> Artificial Sequence

<220>
<223> Description of Artificial Sequence: antisense
      oligonucleotide

<400> 50
tgagctgtgc atgccttc                                                    18

<210> 51
<211> 15
<212> DNA
<213> Artificial Sequence

<220>
<223> Description of Artificial Sequence: antisense
      oligonucleotide

<400> 51
agtcaggagg accag                                                       15

<210> 52
<211> 15
<212> DNA
<213> Artificial Sequence

<220>

<223> Description of Artificial Sequence: antisense
      oligonucleotide

<400> 52
tgggtgccct ggcct                                                    15


<210> 53
<211> 15
<212> DNA
<213> Artificial Sequence

<220>
<223> Description of Artificial Sequence: antisense
      oligonucleotide

<400> 53
catgttaggc aggtt                                                    15


<210> 54
<211> 17
<212> DNA
<213> Artificial Sequence

<220>
<223> Description of Artificial Sequence: antisense
      oligonucleotide

<400> 54
aggcatctcg gagatct                                                  17


<210> 55
<211> 16
<212> DNA
<213> Artificial Sequence

<220>
<223> Description of Artificial Sequence: antisense
      oligonucleotide

<400> 55
aaagtcttca ctctgc                                                   16


<210> 56
<211> 17
<212> DNA
<213> Artificial Sequence

<220>
<223> Description of Artificial Sequence: antisense
      oligonucleotide

<400> 56
aacaagttgt ccagctg                                                  17


<210> 57
<211> 18
<212> DNA

<213> Artificial Sequence

<220>
<223> Description of Artificial Sequence: antisense
      oligonucleotide

<400> 57
gtaaaactgg atcatctc                                                    18


<210> 58
<211> 15
<212> DNA
<213> Artificial Sequence

<220>
<223> Description of Artificial Sequence: antisense
      oligonucleotide

<400> 58
catcacctcc tccag                                                       15


<210> 59
<211> 18
<212> DNA
<213> Artificial Sequence

<220>
<223> Description of Artificial Sequence: antisense
      oligonucleotide

<400> 59
gggtcttcag gttctccc                                                    18


<210> 60
<211> 18
<212> DNA
<213> Artificial Sequence

<220>
<223> Description of Artificial Sequence: antisense
      oligonucleotide

<400> 60
cacggccttg ctcttgtt                                                    18


<210> 61
<211> 18
<212> DNA
<213> Artificial Sequence

<220>
<223> Description of Artificial Sequence: antisense
      oligonucleotide

<400> 61
ttattaaagg cattcttc                                                    18

<210> 62
<211> 18
<212> DNA
<213> Artificial Sequence

<220>
<223> Description of Artificial Sequence: antisense
      oligonucleotide

<400> 62
aagatgtcaa actcactc                                                    18


<210> 63
<211> 18
<212> DNA
<213> Artificial Sequence

<220>
<223> Description of Artificial Sequence: antisense
      oligonucleotide

<400> 63
gtagttgatg aagatgtc                                                    18


<210> 64
<211> 16
<212> DNA
<213> Artificial Sequence

<220>
<223> Description of Artificial Sequence: antisense
      oligonucleotide

<400> 64
gattttggag acctct                                                      16


<210> 65
<211> 16
<212> DNA
<213> Artificial Sequence

<220>
<223> Description of Artificial Sequence: antisense
      oligonucleotide

<400> 65
tcagctatcc cagagc                                                      16


<210> 66
<211> 15
<212> DNA
<213> Artificial Sequence

<220>
<223> Description of Artificial Sequence: antisense
      oligonucleotide

<400> 66

ggctgggtca gctat 15

<210> 67
<211> 18
<212> DNA
<213> Artificial Sequence

<220>
<223> Description of Artificial Sequence: antisense
      oligonucleotide

<400> 67
aaatcgttca cagagaag 18

<210> 68
<211> 18
<212> DNA
<213> Artificial Sequence

<220>
<223> Description of Artificial Sequence: antisense
      oligonucleotide

<400> 68
tctttctaaa tcgttcac 18

<210> 69
<211> 2745
<212> DNA
<213> Artificial Sequence

<220>
<223> Description of Artificial Sequence: antisense
      complementary to mRNA of the human TGF-beta 1

<400> 69
ctgcagcctt gacctcccag gatcaagtga tcctcccacc ttagcctcca gagtagctgg 60
gaccacaggt gtacattttt taaaagtgtt ttgtagagat agggtctcac tatgttaccc 120
aggctggtct caaatgcctg gattcaagta tcctcccatc tctgcctccc aaaagtgcta 180
ggattacagg cgtgagcacc ccgcctggcc tgaactacta tcttttattg tcttcttcac 240
tatcccccac taaagcaggt tcctggtggg caggaactcc tcccttaacc tctctgggct 300
tgtttcctca acctttaaaa tgggtgttat cagagtccct gccatctcag agtgttgcta 360
tggtgactga atgagttcat taatgtaagg cacttcaaca gtgcccaagg tgctcaataa 420
atagatctaa ctacagtagt gttccccact ggtcccctgt gccttgatgc cgggcaaagg 480
aatagtgcag acaggcagga ggaggcagag agggagagag agggagtggg agtgggggaa 540
cgtcagggat ggagacccca ggcaggcgcc caatgacaca gagatccgca gtcctctctc 600
catctttaat ggggccccag gtgggcttgg ggcacggtgt ccttaaatac agcccccatg 660
ggcaaggcag cgggggcggg gcggggtggg gccgggcctg ccggggcggg gcggggcggg 720
gcgggacctc agctgcactt gcaggagcgc acgatcatgt tggacagctg ctccaccttg 780
ggcttgcggc ccacgtagta cacgatgggc agcggctcca gcgcctgcgg cacgcagcac 840
ggcgccgccg aggcgcccgg gttatgctgg ttgtacaggg ccaggacctt gctgtactgc 900
gtgtccaggc tccaaatgta ggggcagggc ccgaggcaga agttggcatg gtagcccttg 960
ggctcgtgga tccacttcca gccgaggtcc ttgcggaagt caatgtacag ctgccgcacg 1020
cagcagttct tctccgtgga gctgaagcaa tagttggtgt ccagggctcg gcggtgccgg 1080
gagctttgca gatgctgggc cctctccagc ggggtggcca tgagaagcag gaaaggccgg 1140
ttcatgccat gaatggtggc caggtcacct cggcggccgg tagtgaaccc gttgatgtcc 1200
acttgcagtg tgttatccct gctgtcacag gagcagtggg cgctaaggcg aaagccctca 1260
atttcccctc cacggctcaa ccactgccgc acaactccgg tgacatcaaa agataaccac 1320
tctggcgagt cgctgggtgc cagcagccgg ttgctgaggt atcgccagga attgttgctg 1380

tatttctggt acagctccac gtgctgctcc acttttaact tgagcctcct cagcagacgc 1440
agctctgccc gggagagcaa cacgggttca ggtaccgctt ctcggagctc tgatgtgttg 1500
aagaacatat atatgctgtg tgtactctgc ttgaacttgt catagatttc gttgtgggtt 1560
tccaccatta gcacgcgggt gacctccttg gcgtagtagt cggcctcagg ctcgggctcc 1620
ggttctgcac tctccccggc cacccggtcg cgggtgctgt tgtacagggc gagcacggcc 1680
tcgggcagcg ggccgggcgg cacctccccc tggctcgggg ggctggcgag ccgcagcttg 1740
gacaggatct ggccgcggat ggcctcgatg cgcttccgct tcaccagctc catgtcgata 1800
gtcttgcagg tggatagtcc cgcggccggc gggccaggcg tcagcaccag tagccacagc 1860
agcggtagca gcagcggcag cagccgcagc ccggagggcg gcatggggga ggcggcgccc 1920
cccggcactg ccgagagcgc gaacagggct ggtgtggtgg ggaggccccg cccctgcagg 1980
ggctgggggt ctcccggcaa aaggtaggag ggcctcgagg gaaagctgag gctcctcagg 2040
gagaagggcg cagtggtgga ggggaggctt ggaccggggg tgtctcagta tcccacggaa 2100
ataacctaga tgggcgcgat ctggtaccag aaggtgggtg gtcttgaata ggggatctgt 2160
ggcaggtcgg agagagatcc gtctcctgga ggagaaaggg tctaggatgc gcgggggctc 2220
aggagacagg ccggggatga aggcggcgtg cagggggtgc gcccgaggtc tggggaaaag 2280
tctttgcggg aggccgggtc ggcgactccc gagggctggt ccggaatggg ggcgcctgag 2340
ggacgccgtg tagggggcag ggagggagca agcgtccccg gcggcaaagg gaggcggtct 2400
ggggtcccca agtcctgcct cctcgcgggg cagcgtcgcg ccaagaggtc cccgcgcctc 2460
cggctcccag cggcaacgga aaagtctcaa aagttttttt cctcttctcc cgaccagctc 2520
gtccctcctc ccgctcctcc tccccctcct ccccgcagtg gcgggggcgg cggcggctcg 2580
tctcagactc tggggcctca ggctgctcct cggcgactcc ttcctccgct ccgggccgag 2640
gccggccccg cgggcggctc agagccgggg ggggtgcccc ggacggggcg tcccccctgc 2700
ccccggccgg ggccctcgct gtctggctgc tccgcggagg gaggt 2745

<210> 70
<211> 1695
<212> DNA
<213> Artificial Sequence

<220>
<223> Description of Artificial Sequence: antisense
      complementary to the mRNA of the human TGF-beta 2

<400> 70
tttaaaaaaa tttgcttctt gtctctctca cttacaaagt aggtgaaatg tagaataagg 60
ccttcaactt tttttgtgtc agatgccagt tttaacaaac agaacacaaa cttccaaagt 120
gtctgaacta gtaccgcctt ttcaaaaatt ttttaacact gatgaaccaa ggctctctta 180
tgttttcttg ttacaagcat catcgttgtc gtcgtcatca tcattatcat catcattgtc 240
attttggtct tgccactttt ccaagaattt tagctgcatt tgcaagactt tacaatcata 300
ttagaaagct gttcaatctt gggtgttttg ccaatgtagt agagaatggt tagaggttct 360
aaatcttggg acacgcagca aggagaagca gatgcttctg gatttatggt attatataag 420
ctcaggaccc tgctgtgctg agtgtctgaa ctccataaat acgggcatgc tccagcacag 480
aagttggcat tgtacccttt gggttcgtgt atccatttcc accctagatc cctcttgaaa 540
tcaatgtaaa gtggacgtag gcagcaatta tcctgcacat ttctaaagca ataggccgca 600
tccaaagcac gcttcttccg ccggttggtc tgttgtgact caagtctgta ggagggcaat 660
aacatt[?]gca ggagatgtgg ggtcttccca ctgttttttt tcctagtgga ctttatagtt 720
ttctgatcac cactggtata tgtggaggtg ccatcaatac ctgcaaatct tgcttctagt 780
tcttcacttt tatttgggat gatgtaatta ttagatggta caaaagtgca gcagggacag 840
tgtaagctta ttttaaatcc caggttcctg tctttatggt gaagccattc atgaacagca 900
tcagttacat cgaaggagag ccattcgcct tctgctcttg ttttcacaac tttgctgtcg 960
atgtagcgct gggttggaga tgttaaatct ttggacttga gaatctgata tagctcaatc 1020
cgttgttcag gcactctggc ttttgggttc tgcaaacgaa agactctgaa ctctgctttc 1080
accaaattgg aagcattctt ctccattgct gagacgtcaa atcgaacaat tctgaagtag 1140
ggtctgtaga aagtgggcgg gatggcattt tcggagggga agaagggcgg catgtctatt 1200
ttgtaaacct ccttggcgta gtactcttcg tcgctcctct cgcgctcgca ggcggccgcc 1260
ctccggctcg ccttctcctg gagcaagtcc ctggtgctgt tgtagatgga aatcacctcc 1320
ggggggactt cctcgggctc aggatagtct tctgggggac tggtgagctt cagcttgctc 1380
aggatctgcc cgcggatcgc ctcgatcctc ttgcgcatga actggtccat atcgagtgtg 1440
ctgcaggtag acaggctgag cgcgaccgtg accagatgca ggatcagaaa agcgctcagc 1500
acacagtagt gcatttttta aaaaagtgga aaaaaaagtt gtttttaaaa gtcagaataa 1560
aaaaaaagaa atcaacaatt ctcaaagtat agatcaagga gagttgtttg gttttttgtt 1620

gttgttgttt gtttttgatg cgaaactttt gcaaacaatc tagtcaatgc ccaacagaaa 1680
aacgtatcct gcttg 1695

<210> 71
<211> 2529
<212> DNA
<213> Artificial Sequence

<220>
<223> Description of Artificial Sequence: antisense
complementary to the mRNA of the human TGF-beta 3

<400> 71
   caggatgccc caaaaatatt tatttataca aagattttga gagtaatatt catacttgtc 60
   tttatacctc agtctatgcg tctgggccca agtcactgtg tggcacatgt cgagcttccc 120
   cgaatgcctc acatgttgtc gcacctgctt ccaggaacac caaatgaaca cagggtcttg 180
   gaggggaagt gggggaagaa cccataatgc cccaaccctg catggaacca caatccagaa 240
   atgtgcatcc tgacctggaa ggcgtctaac caagtgtcca aggggaaata tgatcgaggg 300
   agaggtgaga ggagggaccc agaggcagac aggagagggt tgatttccac cctttcttct 360
   gcgttcagca tatccaaaag gcccaataca gttgatgggc caggaactgc atgacctgga 420
   ttttctccct gtagtgaccc acgatgttaa ttgatgtaga ggacagtttg caaaagtaat 480
   agatttgccc ttaatcccag acagtatgag atacaattct gggactttgt cttcgtaacc 540
   tgtctttaaa aaaaaaaaaa aatgcttgcc ttgtataaca taatccagat tccctagagc 600
   agatgtggta cagcaatgag caaatccaac ctcagatctg aagtgtcttc cagtctggcc 660
   ctgacccagc cattctctgc ccttccttct ccctttaggg tagcccaaat cccattgcca 720
   cacaacatct caacttacca tccctttcct ctatccccat cccctctgtc tgcgtcacag 780
   aaagtctgtg tgttctgaag agttcagcct tcctctaacc aaacccacac tttctttacc 840
   accgtgattc tcagagccag caagaaagaa atgttccaaa aggaaacctc catctcagcc 900
   atttgcccgg agccgaaggt tgtgggctcc aggcctctca gtgaggtttg ttgcttgtgt 960
   gtttcccgag gagcgggcag tcaggcagtg gtggttctct ctcccctctc tctgtcgcac 1020
   gtggggtctc agctacattt acaagacttc accaccatgt tggagagctg ctccactttg 1080
   ggggtcctcc caacatagta caggatggtc aggggctcca ggtcctgggg cacgcagcaa 1140
   ggcgaggcag atgcttcagg gttcagagtg ttgtacagtc ccagcaccgt gctgtgggtt 1200
   gtgtctgcac tgcggaggta tgggcaaggg cctgagcaga agttggcata gtagccctta 1260
   ggttcatgga cccacttcca gcccagatcc tgtcggaagt caatgtagag ggggcgcaca 1320
   cagcagttct cctccaagtt gcggaagcag taattggtgt ccaaagcccg cttcttcctc 1380
   tgacccccct ggcccgggtt gtcgagccgg tgtgggggaa tcatcatgag gattagatga 1440
   gggttgtggt gatccttctg cttcttgagg cgccccagat ctccacggcc atggtcatcc 1500
   tcattgtcca cgcctttgaa tttgatttcc atcacctcgt gaatgttttc caggatatct 1560
   ccattgggct gaaaggtgtg acatggacag tgaatgctga tttctagacc taagttggac 1620
   tctcttctca acagccactc acgcacagtg tcagtgacat caaaggacag ccactcggca 1680
   gtgccccgtg tgggcagatt cttgccaccg atatagcgct gtttggcaat gtgctcatct 1740
   ggccgaagga tctggaagag ctcgatcctc tgctcattcc gcttagagct ggggttgggc 1800
   acccgcaaga cccggaattc tgctcggaat aggttggttc tatttttctc cactgaggac 1860
   acattgaagc ggaaaacctt ggaggtaatt cctttagggc agacagccag ttcgttgtgc 1920
   tccgccagcc cctggatcat gtcgaattta tggatttctt tggcatagta ttccgactcg 1980
   gtgttttcct gggtgcagcc ttcctccctc tccccatgca tctcctccag cagctcccgg 2040
   gtgctgttgt aaagggccag gacctgatag gggacgtggg tcatcaccgt tggctcaggg 2100
   gggctggtga gcctgagctt gctcaagatc tgtcccctaa tggcttccac cctcttcttc 2160
   ttgatgtggc cgaagtccaa ggtggtgcaa gtggacagag agaggctgac cgtggcaaag 2220
   ttcagcaggg ccaggaccac cagagccctt tgcaagtgca tcttcatgtg tgagctggga 2280
   agagaggcca gggggacggc aaggcctgga gaggaagaga ccccagcaga cgtgcagaag 2340
   gagggaggaa aaccaggcgg cctccccaga tcccaaagac tgaggcttgg caagaaggtg 2400
   catgaactca ctgcactgcg agagcttcag gacttccagg aagcgctggc aaccctgagg 2460
   acgaagaagc ggactgtgtg ccttgtagcg ctgggattct tgtccatgtg tctaaacagg 2520
   ttttgctgg 2529

<210> 72
<211> 1259
<212> DNA

<213> Artificial Sequence

<220>
<223> Description of Artificial Sequence: antisense
      complementary to the mRNA of the human
      Interleukine 10

<400> 72
tcaccctatg gaaacagctt aaaaacaggt gaaaataata aatattgaaa aaaattataa 60
tattgggctt ctttctaaat cgttcacaga gaagctcagt aaataaatag aaatgggggt 120
tgaggtatca gaggtaataa atattctata agagaggtac aataaggttt ctcaaggggc 180
tgggtcagct atcccagagc cccagatccg attttggaga cctctaattt atgtcctaga 240
gtctatagag tcgccaccct gatgtctcag tttcgtatct tcattgtcat gtaggcttct 300
atgtagttga tgaagatgtc aaactcactc atggctttgt agatgccttt ctcttggagc 360
ttattaaagg cattcttcac ctgctccacg gccttgctct tgttttcaca gggaagaaat 420
cgatgacagc gccgtagcct cagcctgagg gtcttcaggt tctcccccag ggagttcaca 480
tgcgccttga tgtctgggtc ttggttctca gcttggggca tcacctcctc caggtaaaac 540
tggatcatct cagacaaggc ttggcaaccc aggtaaccct taaagtcctc cagcaaggac 600
tcctttaaca acaagttgtc cagctgatcc ttcatttgaa agaaagtctt cactctgctg 660
aaggcatctc ggagatctcg aagcatgtta ggcaggttgc ctgggaagtg ggtgcagctg 720
ttctcagact gggtgccctg gcctgggctg gccctcaccc cagtcaggag gaccaggcaa 780
cagagcagtg ctgagctgtg catgccttct tttgcaagtc tgtcttgtgg tttggttttg 840
caagagcaac cccctgatgt gtagaccttc acctctctgt ccccctttta tattgtaagc 900
tcagggaggc ctcttcattc attaaaaagc cacaatcaag gtttcccggc acaggatttt 960
ttctgcttag agctcctcct tctctaacct ctctaataaa cttagttttc aatttttgca 1020
tcgtaagcaa aaatgattgg ttgaacatga acttctgcat tacagctatt tttaggatgg 1080
gctacctctc ttagaataat tttttagctt ctcaattaaa aaaagttgat ttcctgggga 1140
gaacagctgt tctgtccgca gaggccctca gctgtgggtt ctcattcgcg tgttcctagg 1200
tcacagtgac gtggacaaat tgcccattcc agaatacaat gggattgaga aataattgg 1259

## Description

This invention is related to effective medicaments in cancer therapy.
The formation of cancer on the one hand is combined with the unwanted growth of tissue and on the other hand is combined with the formation of metastases.
The research in this field has disclosed a lot of mechanisms but still there is no therapy without severe side effects inhibiting metastases or inhibiting tumor progression in solid tumors respectively such as prostate cancer, bladder carcinoma, colon cancer, endometrial cancer, hepatocellular carcinoma, melanoma, leukemia, lymphoma, non-small cell lung cancer (NSCLC) or ovarian cancer.
Tumor derived transforming growth factor beta (TGF-beta) is discussed to play a pivotal role for the malignant progression by inducing metastasis, angiogenesis and tumor cell proliferation. Furtheron it seems to play a central role in the escape mechanism from the immune System in tumor cells.

But the role of TGF beta in the literature is diversely discussed. On the one hand there are experiments indicating that TGP-beta inhibits tumor growth, on the other hand there are experiments that point out that TGF-beta induces cell proliferation, which makes its role in the tumor therapy ambivalent.

An additional point is that TGF-beta has a lot of different subclasses, TGF-beta 1, TGF-beta 2 and TGF-beta 3 whose specific roles in tumor progression are differently discussed, often summarized as TGF-beta and thus sometimes mixed up. The 5 specific role of each TGF-beta subclass, namely TGF-beta 1, TGF-beta 2 and TGF-beta 3 are not so far sufficiently.

EP 1008 649 and EP 0695354 teaches that both, TGF-beta 1 and TGF-beta 2 antisense oligonucleotides can be used for manufacturing of a pharmaceutical composition for the treatment of breast tumor esophageal, gastric carcinomas and skin carcinogenesis. Whereas clinical studies seem to show, that in glioma the TGF-beta seems to play a key role and therefore the TGF-beta 2 antisense oligonucleotides are preferred targets for the treatment of e.g. glioma and breast cancer the Situation is completely different in other tumors.

In prostate cancer for example (Wikström, P., Scand J Urol Nephrol 34 S. 85-94), as well as in some other cancers, there 20 are hints, that TGF-beta levels are increased, but it is not clear if this System can be manipulated for therapeutical purposes.

It is known from interleukine 10 (IL-10) that it plays a central role in the regulation of the immune response. Since it was shown, that some interleukine 10 antisense oligonucleotides can enhance cell-mediated immune response it was important to find potent inhibitors of IL-10 in human to modulate the immune response in a way, that escape mechanisms of tumor cells are compensated, tumor growth is inhibited and the formation of metastases is reduced.

Therefore it was a task of this invention to find appropriate therapeutics for the treatment of tumors that inhibit the unwanted cell proliferation in tumor growth and/or in inhibit the formation of metastases.

One task of this invention is to find therapeutics that inhibit the formation of metastases.
Tumors such as bladder carcinoma, colon cancer, endometrial cancer, hepatocellular carcinoma, leukemia, lymphoma, melanoma, non-small cell lung cancer (NSCLC), ovarian cancer, pancreatic cancer and prostate cancer have poor prognosis and so far no successful therapy is found it.
Therefore it was the task of this invention to find new therapeutics, that have the property specifically inhibiting these tumors.
Another task of this invention is to find new Inhibitors of interleukine 10 (IL-10) for modulating the immune system and appropriate methods of their synthesis.

### Advantages

The mechanism of antisense oligonucleotides seems to work by immuno-modulating effects as well as by direct effects, which could be proofed in experimental studies. This is superior to State of the art inhibition of TGF-beta by e.g. antibodies, since we could show, that cell migration is more effectively inhibited by TGF-beta antisense oligonucleotides than it was possible with TGF-beta antibodies.

The pharmaceutical compositions of this invention have less side effects, show more efficacy, have more bioavailability, show more safety, improved chemical stability.

### Invention

We surprisingly discovered, that antisense oligonucleotides that inhibit the formation of TGF-beta 1, TGF-beta 2, TGF-beta 3, cell-cell adhesion molecules (CAMs), integrins, selectines, metalloproteases (MMPs), their tissue inhibitors (TIMPs) and/or interleukine 10 specific antisense oligonucleotides inhibit the formation of metastases in tumor cell lines.

We further found that antisense oligonucleotides of TGF-beta 1, TGF-beta 3 interleukine 10 inhibit the tumor proliferation of solid tumors such as bladder carcinoma, colon cancer, endometrial cancer, hepatocellular carcinoma, melanoma, non-small cell lung cancer (NSCLC), ovarian cancer, pancreatic cancer and prostate cancer as well as of malignant myeloproliferative diseases such as leukaemia and lymphoma.

Another aspect of this invention are new superior antisense oligonucleotides inhibiting the formation of interleukine 10 (IL-10) and by this modulate the immune response.

Yet another aspect of this invention is the production of IL-10 antisense oligonucleotides.

A further aspect of this invention is the use of interleukine 10 antisense oligonucleotides for the preparation of a pharmaceutical composition.

### Detailed description

### Metastases

In one embodiment the oligonucleotides or active derivatives of this invention are antisense oligonucleotides inhibiting the formation of metastases.

In another embodiment the oligonucleotides or their active derivatives are antisense oligonucleotides inhibiting the synthesis of proteins involved in the formation of metastases.

In a further embodiment the proteins inhibited in their synthesis are selected from the group of e.g. tumor growth factor beta 1 (TGF-beta 1), TGF-beta 2, TGF-beta 3, cell- cell adhesion molecules (CAM), intergrins, selectines, metalloproteases (MMP), their tissue inhibitors (TIMPS) and/or interleukine 10.

One embodiment of the invention is the use of at least one oligonucleotide or its active derivatives of TGF-beta 1, TGF-beta 2, TGF-beta 3 cell-cell adhesion molecules (CAMs), integrins, selectines, metalloproteases (MMPs), their tissue inhibitors (TIMPs) and/or interleukine 10 and/or their active derivatives for the preparation of a pharmaceutical composition for inhibiting the formation of metastases in cancer treatment.

Cell-cell adhesion molecules (CAM) comprise molecules such as intercellular adhesion molecule-1 (ICAM-1), vascular cell adhesion molecule-1 (VCAM-1), endothelial leukocyte adhesion molecule-1 (ELAM-1).

Metalloproteases comprise at least 15 structurally related members also numbered for being identified (MMP-1, MMP-2, etc.) with a broad proteolytic activities against components of the extracellular matrix. Metalloproteases comprise, but are not limited to collagenases, gelatinases, stromelysins and metalloelastases.

Active derivatives of this invention are modifications of the oligonucleotides as described below.

In a preferred embodiment the at least one antisense oligonucleotide or its active derivative for the preparation of a pharmaceutical composition for inhibiting the formation of metastases are sequences identified in the sequence listing under SEQ ID NO 1 to 68, even more preferred are sequences identified in the sequence listing under SEQ ID NO 1, 5, 6, 8, 9, 14, 15, 16, 28, 29, 30, 34, 35, 36, 40 and 42. These oligonucleotides have improved affinity to the target molecule.

In yet another embodiment of this invention the oligonucleotide or its active derivatives are useful in the inhibition of metastases in cancers such as bile duct carcinoma, bladder carcinoma, brain tumor, breast cancer, bronchogenic carcinoma, carcinoma of the kidney, cervical cancer, choriocarcinoma, cystadenocarcinome, cervical carcinoma, colon carcinoma, colorectal carcinoma, embrional carcinoma, endometrial cancer, epithelial carcinoma, esophageal cancer, gallbladder cancer, gastric cancer, head and neck cancer, hepatocellular cancer, liver carcinoma, lung carcinoma, medullary carcinoma, non-small-cell bronchogenic/lung carcinoma, ovarian cancer, pancreas carcinoma, papillary carcinoma, papillary adenocarcinoma, prostate cancer, small intestine carcinoma, rectal cancer, renal cell carcinoma, sebaceous gland carcinoma, skin cancer, small-cell bronchogenic/lung carcinoma, soft tissue cancer, squamous cell carcinoma, testicular carcinoma, uterine cancer, acoustic neuromas, neurofibromas, trachomas, and pyogenic granulomas; pre-malignant tumors, blastoma, Ewing's tumor, craniopharyngloma, ependymoma, medulloblastoma, hemanglioblastoma, medullablastoma, melanoma, mesothelioma, neuroblastoma, neurofibroma, pinealoma, retinoblastoma, retinoblastoma, sarcoma (including angiosarcoma, chondrosarcoma, endothelialsarcoma, fibrosarcoma, gliosarcoma, leiomyosarcoma, liposarcoma, lymphangioandotheliosarcoma, lyphangiosarcoma, melanoma, meningioma, myosarcoma, osteogenic sarcoma, osteosarcoma), seminoma, trachomas, Wilm's tumor.

In a more preferred embodiment the oligonucleotide or its active derivatives are useful in the inhibition of metastases in cancers such as bladder carcinoma, colon cancer, endometrial cancer, hepatocellular carcinoma, melanoma, non-small cell lung cancer (NSCLC), ovarian cancer, pancreatic cancer, prostate cancer, soft tissue cancer.

### Indications

In one embodiment of the invention oligonucleotides or its active derivative are used for the preparation of a pharmaceutical composition for the treatment of bladder carcinoma, colon cancer, endometrial cancer, hepatocellular carcinoma, leukemia, lymphoma, melanoma, non-small cell lung cancer (NSCLC), ovarian cancer, pancreatic cancer, prostate cancer. These oligonucleotides respectively their active derivatives are described in more detail later in this text.

In yet another embodiment the oligonucleotide or its active derivative for the preparation of a pharmaceutical composition for the treatment of the cancers as described above is an antisense oligonucleotide inhibiting the production of tumor growth factor beta 1 (TGF-beta 1), TGF-beta 3 and/or interleukine 10.

In a more preferred embodiment the oligonucleotides or its active derivative for the preparation of a pharmaceutical composition for the treatment of the cancers as described above are antisense oligonucleotides as identified in the sequence listing under SEQ ID NO. 1 to 21 or 49 to 68.

These sequences have especially high affinity to the m-RNA of the respective gene.

Yet another aspect of this invention are new IL-10 antisense-oligonucleotides and their active derivatives identified in the sequence listing under Seq. ID. NO 49 to 68. These oligonucleotides have very high affinity to m-RNA of IL-10 and by this effectively inhibit the synthesis of IL-10.

### Nucleic Acid/Oligonucleotides

One oligonucleotide of this invention is used for the preparation of a pharmaceutical composition for inhibiting the formation of metastases in cancer treatment, oligonucleotides of this invention are also used for the preparation of a pharmaceutical composition for the treatment of bladder carcinoma, colon cancer, endometrial cancer, hepatocellular carcinoma, leukaemia, lymphoma, melanoma, non-small cell lung cancer (NSCLC), ovarian cancer, pancreatic cancer prostate cancer or soft tissue cancer and new oligonucleotides inhibiting the synthesis of IL-10 are part of this invention as well.

The terms oligonucleotide or nucleic acid refer to multiple nucleotides (i.e. molecules comprising a sugar (e.g. ribose or deoxyribose) linked to a phosphate group and to an variable organic base, which is either a substituted pyrimidine (e.g. cytosine (C), thymine (T) or uracil (U)) or a substituted purine (e.g. adenine (A) or guanine (G)) or a modification thereof. As used herein, the terms refer to oligoribonucleotides as well as oligodeoxyribonucleotides. The terms shall also include oligonucleosides (i.e. a oligonucleotide without the phosphate) and any other organic base containing polymer.

Whereas the oligonucleotides of this invention are single stranded, in some embodiments at least parts of the single-stranded nucleic acid is double-stranded. Double-stranded molecules may be more stable in vivo, while single-stranded molecules may have increased activity.

In one embodiment the antisense oligonucleotide is complementary to the whole m-RNA of the gene or any smaller part of the protein which shall be inhibited can be selected, e.g. TGF-beta 1 TGF-beta 2, TGF-beta 3, cell-cell adhesion molecules (CAMs), integrins, selectines, metalloproteases (MMPs), their tissue inhibitors (TIMPs) and/or interleukine 10. In more preferred embodiments the antisense oligonucleotides of this invention have length between about 6 and about 300 nucleotides in yet another embodiment the nucleotides have length of about 7 to about 100 nucleotides respectively from about 8 to about 30 nucleotides, even more preferred from 12 to 24 nucleotides.

Sequences of the respective genes are known to someone skilled in the art, additionally some of them are presented in Example 14.

In still other embodiments, the nucleic acids are not antisense nucleic acids, meaning that they do not function by binding to complementary genomic DNA or RNA species within a cell and thereby inhibiting the function of said genomic DNA or RNA species.

As used herein with respect to linked units of a nucleic acid, "linked" or "linkage" means two entities are bound to one another by any physicochemical means. Any linkage known to those of ordinary skill in the art, covalent or non-covalent, is embraced. Natural linkages, which are those ordinarily found in nature connecting the individual units of a nucleic acid, are most common. The individual units of a nucleic acid may be linked, however, by synthetic or modified linkages.

In one embodiment of this invention at least one phosphate linker of the oligonucleotide is substituted by a peptide. These derivatives of the oligonucleotide is also called a peptide nucleic acid.

In one embodiment the respective ends of this linear polymeric structure can be further joined to form a circular structure. However, open linear structures are generally preferred. Within the oligonucleotides structure, the phosphate groups are commonly referred to as forming the internucleoside backbone of the oligonucleotide. The normal linkage or backbone of RNA and DNA is a 3' to 5' phophodiester linkage.

### Oligonucleotide-Modifications:

Oligonucleotides or nucleic acids includes oligonucleotides having non-naturally-occurring portions with similar function. Such modified or substituted oligonucleotides are often preferred over native forms because of desirable properties such as, for example, enhanced cellular uptake, enhanced affinity for nucleic acid target (e.g. protein), altered intracellular localization and increased stability in the presence of nucleases. Modifications of the oligonucleotides as used herein comprises any chemical modifications of the sugar, the base moiety and/or the internucleoside linkage.

In one embodiment nucleic acids or oligonucleotides with a covalently modified base and/or sugar include for example nucleic acids having backbone sugars which are covalently attached to low molecular weight organic groups other than a hydroxyl group at the 3' and/or 2' position and other than a phosphate group at the 5' position. Thus modified nucleic acids may include a 2'-O-alkylated ribose group. In yet another embodiment modified nucleic acids include sugars such as arabinose instead of ribose. Thus the nucleic acids may be heterogeneous in backbone composition thereby containing any possible combination of polymer units linked together such as peptide-nucleic acids (which have amino acid backbone with nucleic acid bases). In some embodiments the nucleic acids are homogeneous in backbone composition.

The substituted purines and pyrimidines of the nucleic acids include standard purines and pyrimidines such as cytosine as well as base analogs such as C-S propyne substituted bases (Wagner et al., Nature Biotechnology 14:840-844, 1996). Purines and pyrimidines include but are not limited to adenine, cytosine, guanine, thymine, 5-methylcytosine, 2-aminopurine, 2-amino-6-chloropurine, 2,6-diaminopurine, hypoxanthine, and other naturally and non-naturally occurring nucleobases, substituted and unsubstituted aromatic moieties.

The single nucleotides in each oligonucleotide or polynucleotide polymer may contain the same modifications, may contain combinations of these modifications, or may combine these modifications with phosphodiester linkages. Methods of rendering oligonucleotide or polynucleotide polymers nuclease resistant include, but are not limited to, covalently modifying the purine or pyrimidine bases. For example, bases may be methylated, hydroxymethylated, or otherwise substituted (e.g., glycosylated) such that the oligonucleotides or polynucleotides are rendered substantially acid and nuclease resistant.

It is understood to someone skilled in the art, that antisense oligonucleotides in which some nucleotides of the sequence are substituted by another nucleotide or even other spacers, the derivatives of the antisense oligonucleotides of this invention still inhibit the synthesis of proteins such as TGF-beta 1, TGF-beta 2, TGF-beta 3, cell-cell adhesion molecules (CAMs), integrins, selectines, metalloproteases (MMPs), their tissue inhibitors (TIMPs) and/or interleukine 10 etc. In a preferred embodiment about 0,1% to about 50% of the nucleotides are substituted or from about 0,1% to about 10% or from about 0,1% to about 5%.

A spacer as mentioned above can be any chemic substance connecting the at least two parts of the antisense oligonucleotide substituting the space of at least one nucleic acid.

In yet another embodiment at least one T (Thymidine) is substituted by an U (Uracil).

In a preferred embodiment the spacer is another nucleotide or is a sugar such as glucose, ribose etc., an amino acid or one or several units of a polymer such as polypropylene.

In a preferred embodiment, at least one end-block on the oligonucleotide is a biotin, biotin analog, avidin, or avidin analog. These molecules have the ability to both block the degradation of the protected oligonucleotide or polynucleotide and provide means for high affinity attachment of the modified nucleic acids to the solid support. Avidin and biotin derivatives which can be used to prepare the reagents of this invention include streptavidin, succinylated avidin, monomeric avidin, biocytin (biotin-.epsilon.-N-lysine), biocytin hydrazide, amine or sulfhydryl derivatives of 2-iminobiotin and biotinyl-epsilon-aminocaproic acid hydrazide. Additional biotin derivatives, such as biotin-N-hydroxysuccinimide ester, biotinyl-epsilon-aminocaproic acid-N-hydroxysuccinimide ester, sulfosuccinimidyl 6-(biotin amido)hexanoate, N-hydroxysuccinimideiminobiotin, biotinbromoacetylhydrazide, p-diazobenzoyl biocytin and 3-(N-maleimidopropionyl)biocytin, can also be used as end-blocking groups on the polynucleotides of the present invention.

In another embodiment the ring structure of the ribose group of the nucleotides in the modified oligonucleotide or polynucleotide has an oxygen in the ring structure substituted with N-H, N-R (with R being an alkyl or aryl substituent), S and/or methylene.

In a preferred embodiment at least one sugar moiety of the oligonucleotide is a morpholino derivative and the active derivative then is a morpholino oligonucleotide.

In another embodiment two carbones of at least one sugar moiety are linked. The linkage may be with a methoxy group or thereelse. This linker fixes the the sugar moiety in a way that it remains in one specific conformation, which enables e.g. higher selectivity and higher stability of this locked nucleic acids. Locked nucleic acids are described eg. In J. Wengel, Acc. Chem. Res., 120, 5458-5463 (1999) or J. Wengel et al., nucleosides & nucleotides, 18(6&7), S. 1365-1370, herein incorporated by reference.

In yet another embodiment the base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, in particular an aminoethylglycine backbone. The nucleobases are bound directly or indirectly to aza nitrogen atoms of the amide portion of the backbone. Representative United States patents that teach the preparation of PNA compounds include, but are not limited to, U.S. Pat. Nos.: 5,539,082; 5,714,331; and 5,719,262, each of which is herein incorporated by reference. Further teaching of PNA compounds can be found in Nielsen et al., Science, 1991, 254, 1497-1500.

Further modified oligonucleotide backbones include, for example, phosphorothioates, chiral phosphorothioates, phosphorodithioates, phosphotriesters, aminoalkylphosphotriesters, methyl and other alky phosphonates including 3'-alkylene phosphonates and chiral phosphonates, phosphinates, phosphoramidates including 3'-aminophosphoamidate and aminoalkylphosphoramidates, thionophosphoramidates, thionoalkylphosphonates, thionoalkylphosphotriesters, and boranophosphates having norm 3'-5 linkages, 2'-5 linked analogs of these, and those having inverted polarity wherein the adjacent pairs of nucleoside units are linked 3'-5' to 5'-3' or 2'-5' to 5'-2'. Various salts, mixed salts, and free acid forms are also included.

The nucleic acids having backbone modifications useful according to the invention in some embodiments are S- or R-chiral antineoplastic nucleic acids. An "S chiral nucleic acid" as used herein is an nucleic acid wherein at least two nucleotides have a backbone modification forming a chiral center and wherein a plurality of the chiral centers have S chirality. An "R chiral nucleic acid" as used herein is an nucleic acid wherein at least two nucleotides have a backbone modification forming a chiral center and wherein a plurality of the chiral centers have R chirality. The backbone modification may be any type of modification that forms a chiral center. The modifications include but are not limited to phosphorothioate, methylphosphonate, methylphosphorothioate, phosphorodithioate, p-ethoxy, 2'-O-Me and combinations thereof.

Another type of modified backbone, useful according to the invention, is a peptide nucleic acid. The backbone is composed of aminoethyiglycine and supports bases which provide the nucleic acid character. The backbone does not include any phosphate and thus may optionally have no net charge. The lack of charge allows for stronger DNA-DNA binding because the charge repulsion between the two strands does not exist. Additionally, because the backbone has an extra methylene group, the oligonucleotides are enzyme/protease resistant. Peptide nucleic acids can be purchased from various commercial sources, e.g., Perkin Elmer, or synthesized de novo.

### Modification of the 3' /5' end/ irregular modifications

In a further embodiment at least one nucleotide of an oligonucleotide is modified as described in one of the modifications above.

In yet another embodiment both of these modifications of the oligonucleotide are combined.

In preferred embodiments the 1 to about 12 or 1 to about 8 or 1 to about 4 or 1 to about 2 oligonucleotides and/or nucleotide linkages at the 3 ' and/or 5'end of the oligonucleotide are modified as described above.

In yet another embodiment the oligonucleotides of this invention hybridizing with the same target (e.g. TGF-beta 1, TGF-beta 2, TGF-beta 3, cell-cell adhesion molecules (CAMs), integrins, selectines, metalloproteases (MMPs), their tissue inhibitors (TIMPs) and/or interleukine 10) comprising one of the oligonucleotides of the sequence listing but additionally have sequences with about 1 to about 20 nucleotides more preferred from 1 to 15, 1 to 10, 1 to 5, 1 to 3 or 1 to 2 nucleotides on at least one of the 2', 3' and/or 5' end are still within the scope of this invention.

Targets of oligonucleotides mentioned in this invention are known to persons skilled in the art. In a preferred embodiment the targets are selected from the group of m-RNA of TGF-beta 1, TGF-beta 2 and/or TGF-beta 3, cell-cell adhesion molecules (CAMs), integrins, selectines, metalloproteases (MMPs), their tissue inhibitors (TIMPs) and/or interleukine 10. The sequence of the m-RNA of TGF-beta-1 and TGF beta-2 is given in example 6.

### Synthesis

Yet another task of this invention was to find a process of manufacturing the IL-10 antisense oligonucleotides of this invention or its active derivatives.

For the use in the instant invention, the nucleic acids can be synthesized de novo using any of a number of procedures well known in the art. Such compounds are referred to as 'synthetic nucleic acids.' For example, the b-cyanoethyl phosphoramidite method (Beaucage, S. L., and Caruthers, M. H., Tet. Let. 22:1859, 1981); nucleoside H-phosphonate method (Garegg et al., Tet. Let. 27:4051-4054, 1986; Froehler et al., Nucl. Acid. Res. 14:5399-5407, 1986, Garegg et a!, Tet. Let. 27:4055-4058, 1986, Gaffney et al., Tet. Let. 29:2619-2622, 1988). These chemistries can be performed by a variety of automated oligonucleotide synthesizers available in the market.

In a preferred process the IL-10 antisense oligonucleotides of this invention are synthesized by using phosphite triester chemistry growing the nucleotide chain in 3'-5' direction wherein the respective nucleotide is coupled to the first nucleotide that is covalently attached to the solid phase comprising the steps of first cleaving 5'DMT protecting group of the previous nucleotide, then adding the respective nucleotide for chain prolongation, then modifying phosphite groups subsequently cap unreacted 5'-hydroxyl groups and cleaving the oligonucleotides from the solid support and finally working up the synthesis product.

In yet another embodiment nucleic acids are produced on a large scale in plasmids, (see, e.g., Sambrook, et al., Molecular Cloning: A Laboratory Manual, Cold Spring Harbor Laboratory Press, New York, 1989) and separated into smaller pieces or administered as a whole.

In yet another embodiment nucleic acids are prepared from existing nucleic acid sequences (e.g., genomic or cDNA) using techniques known to someone skilled in the art, such as employing restriction enzymes, exonucleases or endonucleases. Nucleic acids prepared in this manner are isolated nucleic acids. The term nucleic acid encompasses both synthetic and isolated antineoplastic nucleic acids.

In another embodiment nucleic acids with modified backbone, such as those having phosphorothioates bonds are synthesized using automated techniques employing, for example, phosphoramidate or H-phosphonate chemistries. Aryl- and alkyl-phosphonates can be made, e.g., as described in U.S. Pat. No. 4,469,863. Alkylphosphotriesters, in which the charged oxygen moiety is alkylated as described in U.S. Pat. No. 5,023,243 and European Patent No. 092,574, can be prepared by automated solid phase synthesis using commercially available reagents.

Methods for making other nucleic acid backbone modifications and substitutions have been described (Uhlmann, E. and Peyman, A., Chem. Rev. 90:544, 1990; Goodchild, J., Bioconjugate Chem. 1:165, 1990).

Descriptions for the synthesis of locked nucleic acids are known to someone skilled in the art, for further details see e.g. Wengel, J., Chem. Res., 120, S. 5458-5463 (1999) or Koch, T., J. Physics Condese Matter 15, S. 1861-1871 (2003).

In one embodiment phosphorothioates are synthesized using automated techniques employing either phosphoramidate or H-phosphonate chemistries. Aryl- and alkyl-phosphonates can be made, e.g., as described in U.S. Pat. No. 4,469,863; and alkylphosphotriesters (in which the charged oxygen moiety is alkylated as described in U.S. Pat. No. 5,023,243 and European Patent No. 092,574) can be prepared by automated solid phase synthesis using commercially available reagents. Methods for making other DNA backbone modifications and substitutions have been described (Uhlmann, E. and Peyman, A., Chem. Rev. 90:544, 1990; Goodchild, J., Bioconjugate Chem. 1:165, 1990).

Other sources of nucleic acids useful according to the invention include standard viral and bacterial vectors, many of which are commercially available. In its broadest sense, a "vector" is any nucleic acid material which is ordinarily used to deliver and facilitate the transfer of nucleic acids to cells. The vector as used herein may be an empty vector or a vector carrying a gene which can be expressed. In the case when the vector is carrying a gene the vector generally transports the gene to the target cells with reduced degradation relative to the extent of degradation that would result in the absence of the vector. In this case the vector optionally includes gene expression sequences to enhance expression of the gene in target cells such as immune cells, but it is not required that the gene be expressed in the cell.

The oligonucleotides and oligonucleotide analogs of this invention can be used in diagnostics, therapeutics and as research reagents and kits. For therapeutic use, the oligonucleotide or oligonucleotide analog is administered to an animal, especially a human, such as are suffering from a illness, more preferred suffering from a tumor and/or metastases.

In a preferred embodiment the IL-10 antisense oligonucleotide are used for the preparation of a pharmaceutical composition for the treatment of cancer and/or inhibiting the formation of metastases.

### Use of antisense oligonucleotides

In a preferred embodiment the TGF-beta 1, TGF-beta 2, TGF-beta 3, cell-cell adhesion molecules (CAMs), integrins, selectines, metalloproteases (MMPs), their tissue inhibitors (TIMPs) and/or interleukine 10 antisense oligonucleotides are particular objects of the present invention. Thus, presenting oligonucleotides and their active derivatives in accordance with the present invention in pharmaceutically acceptable carriers is highly useful. This is especially true for treatment of diseases such as unwanted cancers and metastasis.

In one embodiment the oligonucleotides is useful in the treatment of unwanted cancers or carcinomas such as but not limited to bile duct carcinoma, bladder carcinoma, brain tumor, breast cancer, bronchogenic carcinoma, carcinoma of the kidney, cervical cancer, choriocarcinoma, cystadenocarcinome, embrional carcinoma, epithelial carcinoma, esophageal cancer, cervical carcinoma, colon carcinoma, colorectal carcinoma, endometrial cancer, gallbladder cancer, gastric cancer, head cancer, liver carcinoma, lung carcinoma, medullary carcinoma, neck cancer, non-small-cell bronchogenic/lung carcinoma, ovarian cancer, pancreas carcinoma, papillary carcinoma, papillary adenocarcinoma, prostata cancer, small intestine carcinoma, prostate carcinoma, rectal cancer, renal cell carcinoma, skin cancer, small-cell bronchogenic/lung carcinoma, squamous cell carcinoma, sebaceous gland carcinoma, testicular carcinoma, uterine cancer.

acoustic neuromas, neurofibromas, trachomas, and pyogenic granulomas; pre-malignant tumors, astracytoma, blastoma, Ewing's tumor, craniopharyngloma, ependymoma, medulloblastoma, glioma, hemangloblastoma, Hodgkins-lymphoma, medullablastoma, leukaemia, mesothelioma, neuroblastoma, neurofibroma, non-Hodgkins lymphoma, pinealoma, retinoblastoma, retinoblastoma, sarcoma (including angiosarcoma, chondrosarcoma, endothelialsarcoma, fibrosarcoma, leiomyosarcoma, liposarcoma, lymphangioandotheliosarcoma, lyphangiosarcoma, melanoma, meningioma, myosarcoma, oligodendroglioma, osteogenic sarcoma, osteosarcoma), seminoma, trachomas, Witm's tumor.

### Technology

In one embodiment the oligonucleotides of this invention are administered in aqueous isotonic water solutions is appropriate for intravenous application as well as intratumoral administration. Further pharmaceutical compositions of the present invention comprise oligonucleotides with one or more non-toxic, pharmaceutical acceptable carrier, excipients and/or adjuvants (collectively referred to herein as "carrier material"). The carrier materials are acceptable in the sense of being compatible with the other ingredients of the composition and are not deleterious to the recipient. The pharmaceutical compositions of the present invention can be adapted for administration by any suitable route by selection of appropriate carrier materials and a dosage of oligonucleotides effective for the treatment intended. For example, these compositions can be prepared in a from suitable for administration orally, intravascularyly, intraperitoneally, subcutaneously, intramusculary, rectally or intratumorally. Accordingly the carrier material employed can be a solid or a liquid, or both, and is preferably formulated with the compound as unit -dose composition, for example, a tablet, which can contain from about 1% to about 95%, by weight of oligonucleotides. the concentration of the oligonucleotides in a solution is dependent of the volume being administered. Such pharmaceutical compositions of the invention can be prepared by any of the well known techniques of pharmacy, consisting essentially of admixing the components.

The pharmaceutical composition of this invention is delivered solely or in mixtures. A mixture comprises one or several oligonucleotides of this invention. These at least two substances herein is also referred to as compounds.

In one embodiment the at least two compounds are mixed and pure or in a pharmaceutical acceptable carrier. In yet another embodiment the at least two compounds of the pharmaceutical composition are separate and pure or are separate and in a pharmaceutical acceptable carrier. In one embodiment the at least two components are in the same pharmaceutical acceptable carrier, in yet another embodiment the at least two components are in different pharmaceutical acceptable carriers.

### Forms of administration

Administering the pharmaceutical compositions of the present invention may be accomplished by any means known to the person skilled in the art. Routes of administration include but are not limited to oral, intranasal, intratracheal, ocular, pulmonal, vaginal, rectal, parenteral (e.g. intramuscular, intradermal, intravenous, intratumoral or subcutaneous or direct injection), topical, transdermal.

In one embodiment of a pharmaceutical composition for the treatment of unwanted cancers or carcinomas, the pharmaceutical composition is delivered by means of a biodegradable, polymeric implant or implanted catheters.

The term "pharmaceutical composition" implicates the liquids or substances of this composition are pure and/or combined with pharmaceutical acceptable carriers.

The term "pharmaceutically-acceptable carrier" means one or more compatible solid or liquid filler, diluents or encapsulating substances which are suitable for administration to a human or other vertebrate animal. The term "carrier" denotes an organic or inorganic ingredient, natural or synthetic, with which the active ingredient is combined to facilitate the application. The components of the pharmaceutical compositions also are capable of being commingled with the compounds of the present invention, and with each other, in a manner such that there is no interaction which would substantially impair the desired pharmaceutical efficiency.

Such carriers enable the compounds of the invention to be formulated as tablets, coated tablets, granules, powders, pills, dragees, (micro)capsules, liquids, gels, syrups, slurries, suspensions, emulsions and the like, for oral ingestion by a subject to be treated.

The pharmaceutical compositions may also include granules, powders, tablets, coated tablets, (micro)capsules, suppositories, syrups, emulsions, suspensions, creams, drops, coated onto microscopic gold particles or preparations with protracted release of active compounds, in whose preparation excipients and additives and/or auxiliaries such as disintegrants, binders, coating agents, swelling agents, lubricants, flavorings, sweeteners or solubilizers are customarily used as described above.

For a brief review of present methods for drug delivery, see Langer, Science 249:1527-1533, 1990, which is incorporated herein by reference.

### Oral Forms

For oral administration, the pharmaceutical composition is delivered alone without any pharmaceutical carriers or formulated readily by combining the compound(s) with pharmaceutically acceptable carriers.

In one embodiment pharmaceutical compositions for oral use is obtained as solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatine, gum tragacanth, methyl cellulose, hydroxypropylmethyl-cellulose, sodium carboxymethylcellulose, and/or polyvinylpyrrolidone (PVP).

In yet another embodiment disintegrating agents are added, such as the cross-linked polyvinyl pyrrolidone, agar, or alginic acid or a salt thereof such as sodium alginate. Optionally the oral formulations may also be formulated in saline or buffers for neutralizing internal acid conditions.

In yet another embodiment dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures.

In yet another embodiment dyestuffs or pigments are added to the tablets or dragee coatings for identification or to characterize different combinations of active compound doses.

In another embodiment pharmaceutical preparations which can be used orally include push-fit capsules made of gelatine, as well as soft, sealed capsules made of gelatine and a plasticizer, such as glycerol or sorbitol. In one embodiment the push-fit capsules contains the active ingredient in a mixture with filler such as lactose, binders such as starches, and/or lubricants such as talc or magnesium stearate and, optionally, stabilizers. In another embodiment of the soft capsules, the active compounds are dissolved or suspended in suitable liquids, such as fatty oils, liquid paraffin, or liquid polyethylene glycols. In addition, stabilizers may be added.

In yet another embodiment microspheres formulated for oral administration are used, well know to someone skilled in the art.

The formulations for oral administration are in dosages suitable for such administration.

In yet another embodiment for buccal administration, the compositions may take the form of tablets or lozenges formulated in conventional manner.

### Inhalation

In yet another embodiment for the administration by inhalation, the compounds for use according to the present invention may be conveniently delivered in the form of an aerosol spray, from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g. gelatine for use in an inhaler or insufflator may be formulated containing a powder mix of the compound and a suitable powder base such as lactose or starch.

Suitable pharmaceutical carriers are, for example, aqueous or saline solutions for inhalation, microencapsulated, encochleated, contained in liposomes, nebulized, aerosols.

### Parenteral Application

In yet another embodiment the pharmaceutical acceptable carriers of the compounds for parenteral, intrathecal, intraventricular or intratumoral administration include sterile aqueous solutions which may also contain buffers, diluents and other suitable additives such as, but not limited to, penetration enhancers, carrier compounds and other pharmaceutically acceptable carriers or excipients.

In yet another embodiment for the systemic delivery of the compounds they are in pharmaceutical carriers for parenteral administration by injection (e.g., by bolus injection or continuous infusion). Formulations for injection may be presented in unit dosage form, e.g., in ampoules or in multi-dose containers, with an added preservative. The pharmaceutical compositions take such forms as suspensions, solutions or emulsions in oily or aqueous vehicles, and contain formulatory agents such as suspending, stabilizing and/or dispersing agents.

In one embodiment pharmaceutical carriers for parenteral administration include aqueous solutions of the active compounds in water-soluble form.

In yet another embodiment a suspensions of at least one oligonucleote is prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions comprise substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

In yet another embodiment the active compounds may are in powder form for constitution with a suitable vehicle, e.g., sterile pyrogen-free water, before use or dried onto a sharp object to be scratched into the skin.

### Rectal, vaginal composition

In yet another embodiment the compounds are formulated in rectal or vaginal compositions such as suppositories or retention enemas, e.g., containing conventional suppository bases such as cocoa butter or other glycerides.

### Depot-form:

In yet another embodiment the compounds are formulated as a depot preparation. In one embodiment such long acting formulations are formulated with suitable polymeric or hydrophobic materials (for example as an emulsion in an acceptable oil) or ion exchange resins, or as sparingly soluble derivatives, for example, as a sparingly soluble salt.

In other embodiments delivery systems include time-release, delayed release or sustained release delivery systems. Such systems can avoid repeated administrations of the compounds, increasing convenience to the subject and the physician. Many types of release delivery systems are available and known to those of ordinary skill in the art.

In one embodiment the delivery system includes polymer base systems such as poly(lactide-glycolide), copolyoxalates, polycaprolactones, polyesteramides, polyorthoesters, polyhydroxybutyric acid, and polyanhydrides. Microcapsules of the foregoing polymers containing drugs are described in, for example, U.S. Pat. No. 5,075,109.

In another embodiment the delivery systems include non-polymer systems that are e.g. lipids including sterols such as cholesterol, cholesterol esters and fatty acids or neutral fats such as mono-di-and tri-glycerides; hydrogel release systems; sylastic systems; peptide based systems; wax coatings; compressed tablets using conventional binders and excipients; partially fused implants; and the like.

Specific examples include, but are not limited to: (a) erosional systems in which an agent of the invention is contained in a form within a matrix such as those described in U.S. Pat. No. 4,452,775, 4,675,189, and 5,736,152, and (b) diffusional systems in which an active component permeates at a controlled rate from a polymer such as described in U.S. Pat. No. 3,854,480, 5,133,974 and 5,407,686. In addition, pump-based hardware delivery systems can be used, some of which are adapted for implantation.

In still other embodiments, the antagonist and antineoplastic agent are formulated with GELFOAM, a commercial product consisting of modified collagen fibers that degrade slowly.

### Gel-producer:

In one embodiment the pharmaceutical compositions also comprise suitable solid or gel phase carriers or excipients. Examples of such carriers or excipients include but are not limited to calcium carbonate, calcium phosphate, various sugars, starches, cellulose derivatives, gelatine, and polymers such as polyethylene glycols.

### Salt derivates

In one embodiment the oligonucleotides of this invention are administered neat or in the form of a pharmaceutically acceptable salt. The salts have to be pharmaceutically acceptable, but non-pharmaceutically acceptable salts may conveniently be used to prepare pharmaceutically acceptable salts thereof. Such salts include, but are not limited to, those prepared from the following acids: hydrochloric, hydrobromic, sulphuric, nitric, phosphoric, maleic, acetic, salicylic, p-toluene sulphonic, tartaric, citric, methane sulphonic, formic, malonic, succinic, naphthalene-2-sulphonic, and benzene sulphonic. Also, such salts can be prepared as alkaline metal or alkaline earth salts, such as sodium, potassium or calcium salts of the carboxylic acid group.

### Buffers:

In one embodiment suitable buffering agents include but are not limited to: acetic acid and a salt (1-2% w/v); citric acid and a salt (1-3% w/v); boric acid and a salt (0.5-2.5% w/v); and phosphoric acid and a salt (0.8-2% w/v).

Suitable preservatives include benzalkonium chloride (0.003-0.03% w/v); chlorobutanol (0.3-0.9% w/v); parabens (0.01-0.25% w/v) and thimerosal (0.004-0.02% w/v).

### Topic administration:

In one embodiment the pharmaceutical acceptable carrier for topical administration for the at least two compounds of a pharmaceutical composition according to this invention include transdermal patches, ointments, lotions, creams, gels, drops, suppositories, sprays, liquids and powders. Conventional pharmaceutical carriers, aqueous, powder or oily bases, thickeners and the like may be necessary or desirable. In yet another embodiment coated condoms, gloves and the like are useful.

In yet another embodiment the pharmaceutical compositions also include penetration enhancers in order to enhance the alimentary delivery. Penetration enhancers may be classified as belonging to one of five broad categories, i.e., fatty acids, bile salts, chelating agents, surfactants and non-surfactants (Lee et al., Critical Reviews in Therapeutic Drug Carrier Systems, 1991, 8, 91-192; Muranishi, Critical Reviews in Therapeutic Drug Carrier Systems, 1990, 7, 1-33). One or more penetration enhancers from one or more of these broad categories may be included.

Various fatty acids and their derivatives which act as penetration enhancers include, for example, oleic acid, lauric acid, capric acid, myristic acid, palmitic acid, stearic acid, linoleic acid, linolenic acid, dicaprate, tricaprate, recinleate, monoolein (a.k.a. 1-monooleoyl-rac-glycerol), dilaurin, caprylic acid, arichidonic acid, glyceryl 1-monocaprate, 1-dodecylazacycloheptan-2-one, acylcarnitines, acylcholines, mono- and di-glycerides and physiologically acceptable salts thereof (i.e., oleate, laurate, caprate, myristate, palmitate, stearate, linoleate, etc.) (Lee et al., Critical Reviews in Therapeutic Drug Carrier Systems, 1991, 8:2, 91-192; Muranishi, Critical Reviews in Therapeutic Drug Carrier Systems, 1990, 7:1, 1-33; El-Hariri et al., J. Pharm. Pharmacol., 1992, 44, 651-654). Examples of some presently preferred fatty acids are sodium caprate and sodium laurate, used singly or in combination at concentrations of 0.5 to 5%.

The physiological roles of bile include the facilitation of dispersion and absorption of lipids and fat-soluble vitamins (Brunton, Chapter 38 In: Goodman & Gilman's The Pharmacological Basis of Therapeutics, 9th Ed., Hardman et al., eds., McGraw-Hill, New York, N.Y., 1996, pages 934-935). Various natural bile salts, and their synthetic derivatives, act as penetration enhancers. Thus, the term "bile salt" includes any of the naturally occurring components of bile as well as any of their synthetic derivatives. A presently preferred bile salt is chenodeoxycholic acid (CDCA) (Sigma Chemical Company, St. Louis, Mo.), generally used at concentrations of 0.5 to 2%.

Complex formulations comprising one or more penetration enhancers may be used. For example, bile salts may be used in combination with fatty acids to make complex formulations. Preferred combinations include CDCA combined with sodium caprate or sodium laurate (generally 0.5 to 5%).

### Chelating agents:

In one embodiment additionally chelating agents are used they include, but are not limited to, disodium ethylenediaminetetraacetate (EDTA), citric acid, salicylates (e.g., sodium salicylate, 5-methoxysalicylate and homovanilate), N-acyl derivatives of collagen, laureth-9 and N-amino acyl derivatives of beta-diketones (enamines)(Lee et al., Critical Reviews in Therapeutic Drug Carrier Systems, 1991, 8:2, 92-192; Muranishi, Critical Reviews in Therapeutic Drug Carrier Systems, 1990, 7:1, 1-33; Buur et al., J. Control Rel., 1990, 14, 43-51). Chelating agents have the added advantage of also serving as DNase inhibitors.

### Surfactants

In yet another embodiment additionally surfactants are used. Surfactants include, for example, sodium lauryl sulfate, polyoxyethylene-9-lauryl ether and polyoxyethylene-20-cetyl ether (Lee et al., Critical Reviews in Therapeutic Drug Carrier Systems, 1991, 8:2, 92-191); and perfluorochemical emulsions, such as FC-43 (Takahashi et al., J. Pharm. Pharmacol., 1988, 40, 252-257).

Non-surfactants include, for example, unsaturated cyclic ureas, 1-alkyl- and 1-alkenylazacyclo-alkanone derivatives (Lee et al., Critical Reviews in Therapeutic Drug Carrier Systems, 1991, 8:2, 92-191); and non-steroidal anti-inflammatory agents such as diclofenac sodium, indomethacin and phenylbutazone (Yamashita et al., J. Pharm. Pharmacol., 1987, 39, 621-626).

### Adjuvants

In one embodiment the pharmaceutical compositions of the present invention additionally contain other adjunct components conventionally found in pharmaceutical compositions, at their art-established usage levels. Thus, for example, the compositions may contain additional compatible pharmaceutically-active materials such as, e.g., antipruritics, astringents, local anesthetics or anti-inflammatory agents, or may contain additional materials useful in physically formulating various dosage forms of the composition of present invention, such as dyes, flavoring agents, preservatives, antioxidants, opacifiers, thickening agents and stabilizers. However, such materials, when added, should not unduly interfere with the biological activities of the components of the compositions of the invention.

### Dosage

In the embodiments of oligonucleotides for the preparation of a pharmaceutical composition for inhibiting the formation of metastases in cancer treatment or for the treatment of cancers such as colon colorectal cancer, hepatocellular carcinoma, leukaemia, lymphoma, melanoma, non small cell lung carcinoma, ovarian carcinoma, pancreas carcinoma, prostate carcinoma, soft tissue cancer at least one antisense oligonucleotide is applied in effective amounts. In general, the term "effective amount" of an antisense oligonucleotides refers to the amount necessary or sufficient to realize a desired biologic effect. Specifically, the effective amount is that amount that reduces the rate or inhibits altogether the formation of cancers orcarcinomas respectively inhibits the formation of metastases. For instance, when the subject bears an unwanted cancer, an effective amount is that amount which decreases or eliminates the unwanted cancer. Additionally, an effective amount may be that amount which prevents an increase or causes a decrease in new unwanted cancer and/or reduces the formation of metastases.

The effective amount varies depending upon whether the pharmaceutical composition is used in single or multiple dosages and whether only one or several antisense oligonucleotides are within one pharmaceutical composition.

Dosages given in this writing are for adults. It is quite clear to someone skilled in the art, that these dosages have to be adapted if the human being is a child, a person stressed by a further illness or other circumstances.

The effective dosage is dependent also on the method and means of delivery, which can be localized or systemic. For example, in some applications, as in the treatment of melanoma or ophthalmic cancer the combination preferably is delivered in a topical or ophthalmic carrier.

In one embodiment subject doses of the oligonucleotides described herein typically range from about 0.1 µg to about 10 mg per administration, which depending on the application could be given hourly, daily, weekly, or monthly and any other amount of time therebetween. In yet another embodiment the doses range from about 10 µg to about 5 mg per administration or from about 100 µg to about 1 mg, with 1-10 administrations being spaced hours, days or weeks apart. In some embodiments, however, doses may be used in a range even 2 to 100 fold higher or lower than the typical doses described above.

In one embodiment of this invention the at least one oligonucleotide of a pharmaceutical composition according to this invention is an antisense oligonucleotide inhibiting the production of TGF-beta 1, TGF-beta 3 TGF-beta 1, cell-cell adhesion molecules (CAMs), integrins, selectines, metalloproteases (MMPs), their tissue inhibitors (TIMPs) and/or interleukine 10 is administered in a dose range from about 1 µg/kg/day to about 100 mg/kg/day or from about 10 µg/kg/day to about 10 mg/kg/day or from about 100 µg/kg/day to about 1 mg/kg/day.

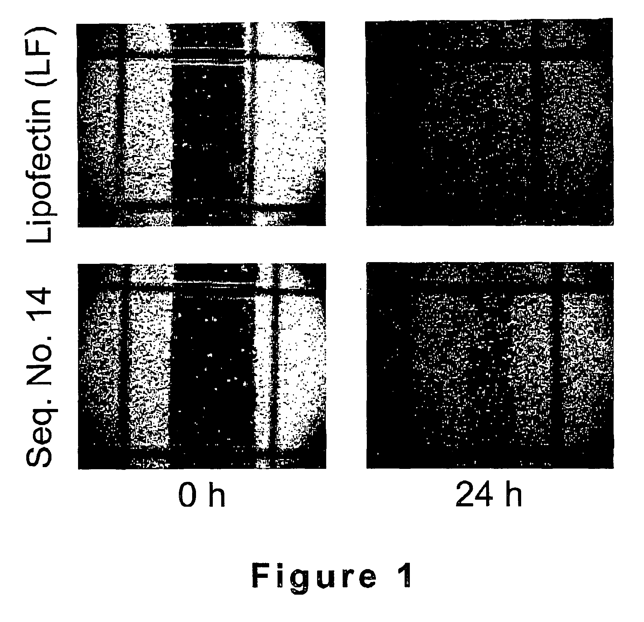

Figure 1 shows the inhibition of prostate cancer cell Line PC-3. The upper two Squares indicate the migration of the control group incubated only with lipofectin. The two Squares below clearly show reduced migration of cells incubated with lipofectin and the antisense oligonucleotide identified in the Sequence listing with Seq. Id. No. 14. The two Squares left hand show the starting conditions. The two Squares at the right hand show the migration after 24 hours, which was clearly inhibited. This indicates a reduced formation of metastases.

### EXAMPLES

### Example 1

### Cell culture conditions for test Systems

The cell lines under test were:

| | |
|---|---|
| Colon cancer | HCT-116 |
| Melanoma | MER 191a, MER 116, MES 100a, |
| NSCLC | SW 900, NCl-H661 |
| Ovarian cancer | EFO-21, Colo 704 |
| Pancreatic cancer | PATU-8902, Hup-T3, Hup-T4 |
| Prostate cancer | PC-3, DU-145 |

Cell lines were obtained from American Type Culture Collection (ATCC) respectively from the German Collection of Microorganisms and Cell Cultures. All cells were cultured as described by the provider.

### Example 2

### Cell-Mediated Cytotoxicity Assay:

For generation of tumor cell supernatants cells were cultured in culture medium supplemented with 1% FCS and 3% Panexin (Pan Biosystems) to reduce the TGF-β amount in the medium. Cells were treated with lipofectin respectively the test substance with lipofectin on two consecutive days or left untreated. Culture supernatants were taken 3 days after the last lipofectin treatment. TGF-beta produced by the tumor cells was activated by 1 N HCl (1:20 dilution) for 10 min at RT. For neutralisation NaOH was added. Human peripheral blood monocytes (PBMC) isolated from healthy donors were incubated with the tumor cell supernatants in the presence of IL-2 (10 ng/ml) for the generation of lymphokine activated killer cells. To block activated TGF-beta 1 a TGF-betal specific antibody (1 µg/ml, R&D Systems) was added. After 3 days, cytotoxic activity of LAK cells was determined in a 4h CARE-LASS assay against the respective cancer cell line as target.

### Example 3

### Proliferation Assay with Lipofectin®

About 75.000 cells per millilitre of the respective tumor cell line were cultured in 12 well flat-bottom microtiter plates in MEM-Dulbecco medium supplemented with 10% fetal calf serum (FCS, Gibco), as recommended by ATCC (American Type Cultur Collection). After 24 hours and after 48 hours the cells were treated with indicated concentration of respective TGF-beta 1 specific antisense oligonucleotides for 6 h. For enhancing the cellular uptake additionally Lipofectin® (Invitrogen, USA) with indicated concentrations was added during this 2 x 6 hours. Between these two periods of 6 h and during the final 3 days cells were treated with 5 µM of above mentioned TGF-beta 1 specific antisense oligonucleotide in the absence of Lipofectin®. Control cells were cultured in pure medium for the same period. Furthermore reference cells were treated with indicated concentration of Lipofectin®.

Finally cell numbers in all samples were quantified by staining the cells with Trypan blue and counting them in a "Neubauer" hemacytometer.

Additionally TGF-beta 1 concentrations in supernatants were measured by TGF-beta 1 Enzym-Linked Immunosorbent Assay (TGF-beta 1 ELISA, R6D Systems, USA) according to the manufacturer instructions. Values from serum-supplemented medium without cells were subtracted to account for background from serum-derived TGF-beta 1.

### Example 4

### Proliferation assay without addition of Lipofectin®

About 25.000-200.000 cells per millilitre, dependent on cell diameter and speed of cell proliferation were cultured in 12 well flat-bottom microtiter plates in MEM-Dulbecco medium supplemented with 10% fetal calf serum (FCS, Gibco), as recommended by ATCC (American Type Cultur Collection). Additionally TGF-beta 1 specific antisense oligonucleotides were added during three days, whereas the control cells remained untreated during the three days.

After three days cell numbers in all samples were quantified by staining them by trypan blue method and counting them in a "Neubauer" hemacytometer.

Additionally TGF-beta 1 concentrations were measured in the supernatants on day 3 with TGF-beta1 Enzym-Linked Immunosorbent Assay (TGF-beta 1 ELISA, Genzyme, Cambridge, MA, USA) according to the manufacturer instructions. Values from serum-supplemented medium without cells were subtracted to account for background from serum-derived TGF-beta 1.

### Example 5

### Scratch Assay

Tumor cells (about 900,000/well) were seeded in 6-well plates. The next day cells were treated once with the test substance and lipofectin respectively with lipofectin for 6h in Optimem (Invitrogen) or left untreated. Then, the confluent monolayer of cells was scratched (start) in a standardized manner with a sterile plastic pipette tip to create a cell-free zone in each well, approximately 1,000µm in width. Afterwards the cells were washed once and incubated at 37°C in standard medium. In vitro migration was documented by photography, and the migration distance was quantified by computer-assisted image analysis using NIH Image 1.6. Each experiment was performed in quadruplicate.

Since migration plays a key role in the formation of metastases this "in vitro" experiment correlates with "in vivo" formation of metastases. Inhibition of "in vitro" migration indicates inhibition of formation of metastases "in vivo".

### Example 6

### Spheroid Migration Model:

A spheroid model of migration was established as described elsewhere (Nygaard et al., 1998). Tumor cells were cultured in tissue culture flasks coated with 2% agar. After 3 days multicellular spheroids were transferred into 96-well plates and left untreated, treated with testsubstance or with recombinant TGF-β2 at 10 ng/ml (R&D Systems). The migration behaviour of the respective cell-line was analyzed by phase contrast microscopy (x10).

Since migration plays a key role in the formation of metastases this "in vitro" experiment correlates with "in vivo" formation of metastases. Inhibition of "in vitro" migration indicates inhibition of formation of metastases "in vivo".

### Example 7

### TGF-β1/β2-ELISA

The amount of TGF-β1 respectively TGF-β2 secreted by tumor cells was determined by ELISA. Briefly, tumor cells (15,000 - 200,000, depending on cell size and cell growth) were seeded into 12-well tissue culture plates and treated with the oligonucleotides under test in the presence of cationic lipid (Lipofectin reagent, Gibco BRL) for 6h in serum-free Optimem medium (Invitrogen) on two consecutive days (final concentrations: AP 11014: 200 nmol/l; Lipofectin: 3 µg/ml) or left untreated. Then, cells were cultured in the presence of 5 µmol/l AP 11014 or left untreated. 72h after the second treatment with lipofectin and the oligonucleotide under test the cell culture supernatants were collected. The amount of TGF-β1/β2 in the supernatants was measured employing a standard TGF-β1-ELISA-Kit respectively TGF-β2 (Quantikine, R&D Systems, USA).

### Example 8 - Colon Cancer

### TGF-β1 Suppression:

Analysed with TGF-β1 specific ELISA, as described in Example 7, 200 nM of phosphorothioate from SEQ ID NO 14 in the presence of 3 µg/ml lipofectin reduced TGF-β1 secretion in colon cancer cell line HCT-116 to about 13,8% compared to untreated control which was set to 100%.

### Cell proliferation:

In the proliferation assay according to example 3 SEQ ID NO 14 in the presence of 3 µg/ml lipofectin reduced proliferation of colon cancer cell line (HCT-116) to about 35% compared to untreated control which was set to 100%.

### Scratch Assay

In the scratch assays according to Example 5 SEQ ID NO 14 significantly inhibits migration of the colon cancer cell line HCT-116 in a concentration of 200 nM in the presence of Lipofectin 3 µg/ml.

### Example 9 - Melanoma

### TGF-beta 1

Analysed with TGF-β1 specific ELISA, as described in Example 7, 400 nM of phosphorothioate from SEQ ID NO 14 in the presence of 1 µg/ml lipofectin reduced TGF-β1 secretion in melanom cancer cell line MER-116 to about 0% compared to untreated control which was set to 100%.

### Inhibition of proliferation

In the proliferation assay according to example 3 SEQ ID NO 14 reduced proliferation of melanoma cell line (MER-116) to about 33% in a concentration of 50 nM an to about 23% in a concentration of 200 nM in the presence of 3 µg/ml lipofectin compared to untreated control which was set to 100%.

### Example 10 - NSCLC

### TGF-β1 Suppression

Analysed with TGF-β1 specific ELISA, as described in Example 7, 200 nM of phosphorothioate from SEQ ID NO 14 in the presence of 3 µg/ml lipofectin reduced TGF-β1 secretion in non small cell lung cancer cell line SW-900 to about 34% and in NCI-H661 to about 38 % compared to untreated control which was set to 100%.

### Cell proliferation:

In the proliferation assay according to example 3 SEQ ID NO 14 reduced proliferation of NSCLC cell line (SW-900) to about 30% in a concentration of 200 nM in the presence of 3 µg/ml lipofectin compared to untreated control which was set to 100%.

### Scratch Assay

In the scratch assays according to Example 5 SEQ ID NO 14 significantly inhibits migration of the NSCLC cancer cell line SW-900 in a concentration of 200 nM and 400 nM in the presence of Lipofectin 6 µg/ml. The migration after 17 h of the cell treated with both concentrations of SEQ ID NO 14 was about 50 µm, whereas the cell incubated with Lipofectin migrated about 75 µm and the control cells about 80 µm. After 24 h the results were about: control 120µm, Lipofectin treated cells: 115µm and SEQ ID NO 14 treated cell about 60µm. Migration after 48 h was about 250 µm for the control and Lipofectin treated cells and about 150 µm for both concentrations of cells treated with SEQ ID NO 14.

### Example 11 - Ovarian cancer

### TGF-beta 1 Suppression

Analysed with TGF-β1 specific ELISA, as described in Example 7, 10 nM of phosphorothioate from SEQ ID NO 14 in the presence of 3 µg/ml lipofectin reduced TGF-β1 secretion in ovarian cancer Colo 704 to about 54% compared to untreated control which was set to 100%.

### Cell proliferation:

TGF-beta 1: In the proliferation assay according to example 3 SEQ ID NO 14 reduced proliferation of ovarian cancer cell line colo 704 to about 54% in a concentration of 50 nM in the presence of 3 µg/ml lipofectin compared to untreated control which was set to 100%.

TGF-beta 2: In the proliferation assay according to example 3 SEQ ID NO 30 reduced proliferation of ovarian cancer cell EFO-21 to about 31% in a concentration of 200 µM and to about 45% in a concentration of 50 nM in the presence of 3 µg/ml lipofectin compared to untreated control which was set to 100%.

### Example 12 - Pancreas:

### TGF-beta 1 Suppression

Analysed with TGF-β1 specific ELISA, as described in Example 7, 10 µM of phosphorothioate from SEQ ID NO 14 reduced TGF-β1 secretion in pancreatic cancer Hup T3 to about 74% compared to untreated control which was set to 100%.

### TGF-beta 2 Suppression

Analysed with TGF-beta 2 specific ELISA, as described in Example 7, 200 nM of phosphorothioate from SEQ ID NO 30 in the presence of 3 µg/ml lipofectin reduced TGF-β2 secretion in pancreatic cancer cell line Hup-T3 to about 2% and in PATU-8902 to about 10% compared to untreated control which was set to 100%.

### Cell Proliferation

In the proliferation assay according to example 3 SEQ ID NO 30 reduced proliferation of pancreatic cancer cell line Hup-T3 to about 1% in a concentration of 200 nM in the presence of 3 µg/ml lipofectin compared to untreated control which was set to 100%, in the cell line PATU-8902 proliferation was reduced to about 10%.

### Cell Proliferation

In the proliferation assay according to example 4 SEQ ID NO 14 reduced proliferation of pancreatic cancer cell line Hup-T3 to about 13% in a concentration of 10 µM in the presence of 3 µg/ml lipofectin compared to untreated control which was set to 100%, in the cell line PATU-8902 proliferation was reduced to about 10%.

### Cell Migration

Migration of a pancreatic cell line PATU- 8902 was measured according to the protocol of example 6 treated with SEQ ID NO 30 in a concentration of 5 µMol/l was nearly completely inhibited for about 65 h, compared to the untreated reference whereas the diameter of the sphere of control cells increased about 1000 µm during the same time.

In the same experiment human TGF-beta 2 antibodies were tested which nearly showed no effect, which indicates that the inhibition of migration is highly specific for antisense oligonucleotides and do not only correlate with antagonizing TGF-beta.

### Example 13 - Prostate Cancer

### TGF-beta 1 Suppression

Analysed with TGF-β1 specific ELISA, as described in Example 7, 200 nM of phosphorothioate from SEQ ID NO 14 in the presence of 3 µg/ml lipofectin reduced TGF-β1 secretion in prostate cancer cell line PC-3 to about 36% and in DU-145 to about 57% compared to untreated control which was set to 100%.

### Cell proliferation:

In the proliferation assay according to example 3 SEQ ID NO 14 reduced proliferation of prostate carcinoma cell line PC-3 to about 74% in a concentration of 200 nM in the presence of 3 µg/ml lipofectin compared to untreated control which was set to 100%.

### Scratch Assay

In the scratch assays according to Example 5 SEQ ID NO 14 significantly inhibits migration of the prostate cancer cell line PC-3 in a concentration of 400 nM in the presence of Lipofectin 6 µg/ml. The migration after 17 h of the cell treated with SEQ ID NO 14 was about 37 µm, whereas the cell incubated with Lipofectin migrated about 140 µm and the control cells about 165 µm. After 24 h the results were about: control 288 µm, Lipofectin treated cells: 213 µm and SEQ ID NO 14 treated cell about 60 µm. Migration after 48 h was about 366 µm for the control, 328 µm for Lipofectin treated cells and about 150 µm for cells treated with SEQ ID NO 14.

### Example 14- m RNA of genes TGF-beta 1, TGF-beta 2, TGF-beta 3 IL-10

Antisense complementary to m-RNA of the human transforming growth factor beta 1 (TGF-beta 1):

CTGCAGCCTTGACCTCCCAGGATCAAGTGATCCTCCCACCTTAGCCTCCAGAGTAGCTGGGACCACAGGTGTACA
TTTTTTAAAAGTGTTTTGTAGAGATAGGGTCTCACTATGTTACCCAGGCTGGTCTCAAATGCCTGGATTCAAGTA

TCCTCCCATCTCTGCCTCCCAAAAGTGCTAGGATTACAGGCGTGAGCACCCCGCCTGGCCTGAACTACTATCTTT
TATTGTCTTCTTCACTATCCCCCACTAAAGCAGGTTCCTGGTGGGCAGGAACTCCTCCCTTAACCTCTCTGGGCT
TGTTTCCTCAACCTTTAAAATGGGTGTTATCAGAGTCCCTGCCATCTCAGAGTGTTGCTATGGTGACTGAATGAG
TTCATTAATGTAAGGCACTTCAACAGTGCCCAAGGTGCTCAATAAATAGATCTAACTACAGTAGTGTTCCCCACT
GGTCCCCTGTGCCTTGATGCCGGGCAAAGGAATAGTGCAGACAGGCAGGAGGAGGCAGAGAGGGAGAGAGAGGGA
GTGGGAGTGGGGGAACGTCAGGGATGGAGACCCCAGGCAGGCGCCCAATGACACAGAGATCCGCAGTCCTCTCTC
CATCTTTAATGGGGCCCCAGGTGGGCTTGGGGCACGGTGTCCTTAAATACAGCCCCCATGGGCAAGGCAGCGGGG
GCGGGGCGGGGTGGGGCCGGGCCTGCCGGGGCGGGGCGGGGCGGGGCGGGACCTCAGCTGCACTTGCAGGAGCGC
ACGATCATGTTGGACAGCTGCTCCACCTTGGGCTTGCGGCCCACGTAGTACACGATGGGCAGCGGCTCCAGCGCC
TGCGGCACGCAGCACGGCGCCGCCGAGGCGCCCGGGTTATGCTGGTTGTACAGGGCCAGGACCTTGCTGTACTGC
GTGTCCAGGCTCCAAATGTAGGGGCAGGGCCCGAGGCAGAAGTTGGCATGGTAGCCCTTGGGCTCGTGGATCCAC
TTCCAGCCGAGGTCCTTGCGGAAGTCAATGTACAGCTGCCGCACGCAGCAGTTCTTCTCCGTGGAGCTGAAGCAA
TAGTTGGTGTCCAGGGCTCGGCGGTGCCGGGAGCTTTGCAGATGCTGGGCCCTCTCCAGCGGGGTGGCCATGAGA
AGCAGGAAAGGCCGGTTCATGCCATGAATGGTGGCCAGGTCACCTCGGCGGCCGGTAGTGAACCCGTTGATGTCC
ACTTGCAGTGTGTTATCCCTGCTGTCACAGGAGCAGTGGGCGCTAAGGCGAAAGCCCTCAATTTCCCCTCCACGG
CTCAACCACTGCCGCACAACTCCGGTGACATCAAAAGATAACCACTCTGGCGAGTCGCTGGGTGCCAGCAGCCGG
TTGCTGAGGTATCGCCAGGAATTGTTGCTGTATTTCTGGTACAGCTCCACGTGCTGCTCCACTTTTAACTTGAGC
CTCCTCAGCAGACGCAGCTCTGCCCGGGAGAGCAACACGGGTTCAGGTACCGCTTCTCGGAGCTCTGATGTGTTG
AAGAACATATATATGCTGTGTGTACTCTGCTTGAACTTGTCATAGATTTCGTTGTGGGTTTCCACCATTAGCACG
CGGGTGACCTCCTTGGCGTAGTAGTCGGCCTCAGGCTCGGGCTCCGGTTCTGCACTCTCCCCGGCCACCCGGTCG
CGGGTGCTGTTGTACAGGGCGAGCACGGCCTCGGGCAGCGGGCCGGGCGGCACCTCCCCCTGGCTCGGGGGGCTG
GCGAGCCGCAGCTTGGACAGGATCTGGCCGCGGATGGCCTCGATGCGCTTCCGCTTCACCAGCTCCATGTCGATA
GTCTTGCAGGTGGATAGTCCCGCGGCCGGCGGGCCAGGCGTCAGCACCAGTAGCCACAGCAGCGGTAGCAGCAGC
GGCAGCAGCCGCAGCCCGGAGGGCGGCATGGGGGAGGCGGCGCCCCCCGGCACTGCCGAGAGCGCGAACAGGGCT
GGTGTGGTGGGGAGGCCCCGCCCCTGCAGGGGCTGGGGGTCTCCCGGCAAAAGGTAGGAGGGCCTCGAGGGAAAG
CTGAGGCTCCTCAGGGAGAAGGGCGCAGTGGTGGAGGGGAGGCTTGGACCGGGGGTGTCTCAGTATCCCACGGAA
ATAACCTAGATGGGCGCGATCTGGTACCAGAAGGTGGGTGGTCTTGAATAGGGGATCTGTGGCAGGTCGGAGAGA
GATCCGTCTCCTGGAGGAGAAAGGGTCTAGGATGCGCGGGGGCTCAGGAGACAGGCCGGGGATGAAGGCGGCGTG
CAGGGGGTGCGCCCGAGGTCTGGGGAAAAGTCTTTGCGGGAGGCCGGGTCGGCGACTCCCGAGGGCTGGTCCGGA
ATGGGGGCGCCTGAGGGACGCCGTGTAGGGGGCAGGGAGGGAGCAAGCGTCCCCGGCGGCAAAGGGAGGCGGTCT
GGGGTCCCCAAGTCCTGCCTCCTCGCGGGGCAGCGTCGCGCCAAGAGGTCCCCGCGCCTCCGGCTCCCAGCGGCA
ACGGAAAAGTCTCAAAAGTTTTTTTCCTCTTCTCCCGACCAGCTCGTCCCTCCTCCCGCTCCTCCTCCCCCTCCT
CCCCGCAGTGGCGGGGGCGGCGGCGGCTCGTCTCAGACTCTGGGGCCTCAGGCTGCTCCTCGGCGACTCCTTCCT
CCGCTCCGGGCCGAGGCCGGCCCCGCGGGCGGCTCAGAGCCGGGGGGGGTGCCCCGGACGGGGCGTCCCCCCTGC
CCCCGGCCGGGGCCCTCGCTGTCTGGCTGCTCCGCGGAGGGAGGT

Antisense complementary to m-RNA of the human transforming growth factor beta 2 (TGF-beta2):

TTTAAAAAAATTTGCTTCTTGTCTCTCTCACTTACAAAGTAGGTGAAATGTAGAATAAGGCCTTCAACTTTTTTT
GTGTCAGATGCCAGTTTTAACAAACAGAACACAAACTTCCAAAGTGTCTGAACTAGTACCGCCTTTTCAAAAATT
TTTTAACACTGATGAACCAAGGCTCTCTTATGTTTTCTTGTTACAAGCATCATCGTTGTCGTCGTCATCATCATT

ATCATCATCATTGTCATTTTGGTCTTGCCACTTTTCCAAGAATTTAGCTGCATTTGCAAGACTTTACAATCATA
TTAGAAAGCTGTTCAATCTTGGGTGTTTTGCCAATGTAGTAGAGAATGGTTAGAGGTTCTAAATCTTGGGACACG
CAGCAAGGAGAAGCAGATGCTTCTGGATTTATGGTATTATATAAGCTCAGGACCCTGCTGTGCTGAGTGTCTGAA
CTCCATAAATACGGGCATGCTCCAGCACAGAAGTTGGCATTGTACCCTTTGGGTTCGTGTATCCATTTCCACCCT
AGATCCCTCTTGAAATCAATGTAAAGTGGACGTAGGCAGCAATTATCCTGCACATTTCTAAAGCAATAGGCCGCA
TCCAAAGCACGCTTCTTCCGCCGGTTGGTCTGTTGTGACTCAAGTCTGTAGGAGGGCAATAACATTAGCAGGAGA
TGTGGGGTCTTCCCACTGTTTTTTTCCTAGTGGACTTTATAGTTTTCTGATCACCACTGGTATATGTGGAGGTG
CCATCAATACCTGCAAATCTTGCTTCTAGTTCTTCACTTTTATTTGGATGATGTAATTATTAGATGGTACAAAA
GTGCAGCAGGGACAGTGTAAGCTTATTTTAAATCCCAGGTTCCTGTCTTTATGGTGAAGCCATTCATGAACAGCA
TCAGTTACATCGAAGGAGAGCCATTCGCCTTCTGCTCTTGTTTTCACAACTTTGCTGTCGATGTAGCGCTGGGTT
GGAGATGTTAAATCTTTGGACTTGAGAATCTGATATAGCTCAATCCGTTGTTCAGGCACTCTGGCTTTTGGGTTC
TGCAAACGAAAGACTCTGAACTCTGCTTTCACCAAATTGGAAGCATTCTTCTCCATTGCTGAGACGTCAAATCGA
ACAATTCTGAAGTAGGGTCTGTAGAAAGTGGGCGGGATGGCATTTTCGGAGGGGAAGAAGGGCGGCATGTCTATT
TTGTAAACCTCCTTGGCGTAGTACTCTTCGTCGCTCCTCTCGCGCTCGCAGGCGGCCGCCCTCCGGCTCGCCTTC
TCCTGGAGCAAGTCCCTGGTGCTGTTGTAGATGGAAATCACCTCCGGGGGGACTTCCTCGGGCTCAGGATAGTCT
TCTGGGGGACTGGTGAGCTTCAGCTTGCTCAGGATCTGCCCGCGGATCGCCTCGATCCTCTTGCGCATGAACTGG
TCCATATCGAGTGTGCTGCAGGTAGACAGGCTGAGCGCGACCGTGACCAGATGCAGGATCAGAAAAGCGCTCAGC
ACACAGTAGTGCATTTTTTAAAAAAGTGGAAAAAAAAGTTGTTTTTAAAAGTCAGAATAAAAAAAAAGAAATCAA
CAATTCTCAAAGTATAGATCAAGGAGAGTTGTTTGGTTTTTTGTTGTTGTTTGTTTTTGATGCGAAACTTTT
GCAAACAATCTAGTCAATGCCCAACAGAAAAACGTATCCTGCTTG

Antisense complementary to m-RNA of the human transforming growth factor beta 3 (TGF-beta 3)

CAGGATGCCCCAAAAATATTTATTTATACAAAGATTTTGAGAGTAATATTCATACTTGTCTTTATACCTCAGTCT
ATGCGTCTGGGGCCAAGTCACTGTGTGGCACATGTCGAGCTTCCCCGAATGCCTCACATGTTGTCGCACCTGCTT
CCAGGAACACCAAATGAACACAGGGTCTTGGAGGGAAGTGGGGAAGAACCCATAATGCCCCAACCCTGCATGG
AACCACAATCCAGAAATGTGCATCCTGACCTGGAAGGCGTCTAACCAAGTGTCCAAGGGGAAATATGATCGAGGG
AGAGGTGAGAGGAGGGACCCAGAGGCAGACAGGAGAGGGTTGATTTCCACCCTTTCTTCTGCGTTCAGCATATCC
AAAAGGCCCAATACAGTTGATGGGCCAGGAACTGCATGACCTGGATTTTCTCCCTGTAGTGACCCACGATGTTAA
TTGATGTAGAGGACAGTTTGCAAAAGTAATAGATTTGCCCTTAATCCCAGACAGTATGAGATACAATTCTGGGAC
TTTGTCTTCGTAACCTGTCTTTAAAAAAAAAAAAAAATGCTTGCCTTGTATAACATAATCCAGATTCCCTAGAGC
AGATGTGGTACAGCAATGAGCAAATCCAACCTCAGATCTGAAGTGTCTTCCAGTCTGGCCCTGACCCAGCCATTC
TCTGCCCTTCCTTCTCCCTTTAGGGTAGCCCAAATCCCATTGCCACACAACATCTCAACTTACCATCCCTTTCCT
CTATCCCCATCCCCTCTGTCTGCGTCACAGAAAGTCTGTGTGTTCTGAAGAGTTCAGCCTTCCTCTAACCAAACC
CACACTTTCTTTACCACCGTGATTCTCAGAGCCAGCAAGAAAGAAATGTTCCAAAAGGAAACCTCCATCTCAGCC
ATTTGCCCGGAGCCGAAGGTTGTGGGCTCCAGGCCTCTCAGTGAGGTTTGTTGCTTGTGTGTTTCCCGAGGAGCG
GGCAGTCAGGCAGTGGTGGTTCTCTCTCCCCTCTCTCTGTCGCACGTGGGGTCTCAGCTACATTTACAAGACTTC
ACCACCATGTTGGAGAGCTGCTCCACTTTGGGGGTCCTCCCAACATAGTACAGGATGGTCAGGGGCTCCAGGTCC
TGGGGCACGCAGCAAGGCGAGGCAGATGCTTCAGGGTTCAGAGTGTTGTACAGTCCCAGCACCGTGCTGTGGGTT
GTGTCTGCACTGCGGAGGTATGGGCAAGGGCCTGAGCAGAAGTTGGCATAGTAGCCCTTAGGTTCATGGACCCAC
TTCCAGCCCAGATCCTGTCGGAAGTCAATGTAGAGGGGGCGCACACAGCAGTTCTCCTCCAAGTTGCGGAAGCAG

TAATTGGTGTCCAAAGCCCGCTTCTTCCTCTGACCCCCCTGGCCCGGGTTGTCGAGCCGGTGTGGGGAATCATC
ATGAGGATTAGATGAGGGTTGTGGTGATCCTTCTGCTTCTTGAGGCGCCCCAGATCTCCACGGCCATGGTCATCC
TCATTGTCCACGCCTTTGAATTTGATTTCCATCACCTCGTGAATGTTTTCCAGGATATCTCCATTGGGCTGAAAG
GTGTGACATGGACAGTGAATGCTGATTTCTAGACCTAAGTTGGACTCTCTTCTCAACAGCCACTCACGCACAGTG
TCAGTGACATCAAAGGACAGCCACTCGGCAGTGCCCCGTGTGGGCAGATTCTTGCCACCGATATAGCGCTGTTTG
GCAATGTGCTCATCTGGCCGAAGGATCTGGAAGAGCTCGATCCTCTGCTCATTCCGCTTAGAGCTGGGGTTGGGC
ACCCGCAAGACCCGGAATTCTGCTCGGAATAGGTTGGTTCTATTTTTCTCCACTGAGGACACATTGAAGCGGAAA
ACCTTGGAGGTAATTCCTTTAGGGCAGACAGCCAGTTCGTTGTGCTCCGCCAGCCCCTGGATCATGTCGAATTTA
TGGATTTCTTTGGCATAGTATTCCGACTCGGTGTTTTCCTGGGTGCAGCCTTCCTCCCTCTCCCCATGCATCTCC
TCCAGCAGCTCCCGGGTGCTGTTGTAAAGGGCCAGGACCTGATAGGGGACGTGGGTCATCACCGTTGGCTCAGGG
GGGCTGGTGAGCCTGAGCTTGCTCAAGATCTGTCCCCTAATGGCTTCCACCCTCTTCTTCTTGATGTGGCCGAAG
TCCAAGGTGGTGCAAGTGGACAGAGAGAGGCTGACCGTGGCAAAGTTCAGCAGGGCCAGGACCACCAGAGCCCTT
TGCAAGTGCATCTTCATGTGTGAGCTGGGAAGAGAGGCCAGGGGGACGGCAAGGCCTGGAGAGGAAGAGACCCCA
GCAGACGTGCAGAAGGAGGGAGGAAAACCAGGCGGCCTCCCCAGATCCCAAAGACTGAGGCTTGGCAAGAAGGTG
CATGAACTCACTGCACTGCGAGAGCTTCAGGACTTCCAGGAAGCGCTGGCAACCCTGAGGACGAAGAAGCGGACT
GTGTGCCTTGTAGCGCTGGGATTCTTGTCCATGTGTCTAAACAGGTTTTGCTGG

Antisense of m-RNA of human Interleukine 10

TCACCCTATGGAAACAGCTTAAAAACAGGTGAAAATAATAAATATTGAAAAAAATTATAATATTGGGCTTCTTTC
TAAATCGTTCACAGAGAAGCTCAGTAAATAAATAGAAATGGGGGTTGAGGTATCAGAGGTAATAAATATTCTATA
AGAGAGGTACAATAAGGTTTCTCAAGGGGCTGGGTCAGCTATCCCAGAGCCCCAGATCCGATTTTGGAGACCTCT
AATTTATGTCCTAGAGTCTATAGAGTCGCCACCCTGATGTCTCAGTTTCGTATCTTCATTGTCATGTAGGCTTCT
ATGTAGTTGATGAAGATGTCAAACTCACTCATGGCTTTGTAGATGCCTTTCTCTTGGAGCTTATTAAAGGCATTC
TTCACCTGCTCCACGGCCTTGCTCTTGTTTTCACAGGGAAGAAATCGATGACAGCGCCGTAGCCTCAGCCTGAGG
GTCTTCAGGTTCTCCCCCAGGGAGTTCACATGCGCCTTGATGTCTGGGTCTTGGTTCTCAGCTTGGGGCATCACC
TCCTCCAGGTAAAACTGGATCATCTCAGACAAGGCTTGGCAACCCAGGTAACCCTTAAAGTCCTCCAGCAAGGAC
TCCTTTAACAACAAGTTGTCCAGCTGATCCTTCATTTGAAAGAAAGTCTTCACTCTGCTGAAGGCATCTCGGAGA
TCTCGAAGCATGTTAGGCAGGTTGCCTGGAAGTGGGTGCAGCTGTTCTCAGACTGGGTGCCCTGGCCTGGGCTG
GCCCTCACCCCAGTCAGGAGGACCAGGCAACAGAGCAGTGCTGAGCTGTGCATGCCTTCTTTTGCAAGTCTGTCT
TGTGGTTTGGTTTTGCAAGAGCAACCCCCTGATGTGTAGACCTTCACCTCTCTGTCCCCCTTTTATATTGTAAGC
TCAGGGAGGCCTCTTCATTCATTAAAAAGCCACAATCAAGGTTTCCCGGCACAGGATTTTTTCTGCTTAGAGCTC
CTCCTTCTCTAACCTCTCTAATAAACTTAGTTTTCAATTTTTGCATCGTAAGCAAAAATGATTGGTTGAACATGA
ACTTCTGCATTACAGCTATTTTTAGGATGGGCTACCTCTCTTAGAATAATTTTTTAGCTTCTCAATTAAAAAAAG
TTGATTTCCTGGGGAGAACAGCTGTTCTGTCCGCAGAGGCCCTCAGCTGTGGGTTCTCATTCGCGTGTTCCTAGG
TCACAGTGACGTGGACAAATTGCCCATTCCAGAATACAATGGGATTGAGAAATAATTGG

### Example 15 -Synthesis of oligonucleotides

On method for synthesizing oligondesoxy-nucleotides is performed by stepwise 5 addition of protected nucleosides using phosphite triester chemistry. The first nucleotide is introduced as 5'-dimethoxytrityl-deoxyadenosine(N⁴benzoyl)-N N'diisopropyl-2-cyanoethyl phosphoramidite (0 1 M); C is introduced by a 5'dimethoxytrityl-deoxycytidine (N⁴benzoyl)-N, N'-diisopropyl-2-cyanoethyl phosphoramidite; G is introduced as 5'-dimethoxytrityl-deoxyguanosine(N⁸isobutyryl)-N, N'diisopropyl-2-cyanoethyl phosphoramidite and the T was introduced as 5'-dimethoxytrityl-deoxythymidine-N, N'-diisopropyl-2-cyanoethyl phosphoramidite. The nucleosides were preferably applied in 0.1 M concentration dissolved in acetonitril.

Synthesis is performed on controlled pore glass particles of about 150 Fm diameter (pore diameter about 500 Â) to which the most 3 nucleoside is covalently attached via a long chain alkylamin linker (average loading about 30 Fmol/g solid support).

The solid support is loaded into a cylindrical synthesis column capped on both ends with filters which permit adequate flow of reagents but hold back the solid synthesis support. Reagents are delivered and withdrawn from the synthesis column using positive pressure of inert gas The nucleotides were added to the growing oligonucleotide chain in 3' → 5 ' direction. Each nucleotide was coupled using one round of the following synthesis cycle.

Cleaving 5'DMT (dimethoxytrityl) protecting group of the previous nucleotide with 3-chloroacetic acid in dichloromethane followed by washing the column with anhydrous acetonitrile. Then simultaneously one of the bases in form of their protected derivative depending on the sequence is added plus tetrazole in acetonitrile. After reaction the reaction mixture has been withdrawn and the phosphite is oxidized with a mixture of sulfur (S₈) in carbon disulfid/pyridine/triethylamine. After the oxidation reaction the mixture is withdrawn and the column was washed with acetonitrile. The unreacted 5'-hydroxyl groups are capped with simultaneous addition of 1 -methyltmidazole and acetic anhydryide/lutidine/tetrahydrofuran. Thereafter the synthesis column is washed with acetonitrile and the next cycle was started.

The work up procedure and purification of the synthesis products occurs as follows:

After the addition of the last nucleotide the deoxynucleotides were cleaved from the solid support by incubation in ammonia solution. Exoxyclic base protecting groups are removed by further incubation in ammonia. Then the ammonia is evaporated under vacuum. Full-length synthesis products still bearing the 5 ' DMT protecting group are separated from shorter failure contaminants using reverse phase high performance liquid chromatography on silica C₁₈ stationary phase. Eluents from the product peak are collected dried under vacuum and the 5'-DMT protecting group cleaved by incubation in acetic acid which is evaporated thereafter under vacuum. The synthesis products are solubilized in the deionized water and extracted three times with diethylether. Then the products are dried in vacuo. Another HPLC-AX chromatography is performed and the eluents from the product peak are dialysed against excess of Tris-buffer and then dialysed against deionized water. The final products are lyophilized and stored dry.

### Example 16

Cell mediated cytotoxicity assays were performed according to protocol of Example 2 with phosphorthioated antisense oligonucleotides of TGF beta 2: SEQ ID NO 30 respectively TGF-beta 1: SEQ ID NO 14.

TGF-beta 2 - pancreatic cancer cell line PA-TU-8902 Surprisingly cell mediated cytotoxicity inhibited by high levels of TGF-beta 2, was nearly completely restored in the pancreatic cancer cell linePA-TU-8902 by 200 nM of SEQ ID NO 30 in the presence of 3/µg/ml lipofectin. The test was consolidated by different ratios of peripheral blood mononuclear cells (PBMCs) to tumor cells (10:1, 5:1, 2,5:1). The respective restoration of the cell mediated cytotoxicity was 80%, 88% and 100%. Results were taken from triplicates. Comparable results were found in non-small cell lung carcinoma cell line K562, colon cancer cell line HCT-116.

### TGF-beta 1 - colon cancer cell line K562

Cell mediated cytotoxicity inhibited by high levels of TGF-beta 1, was completely restored in the colon cancer cell line K562 by 400 nM of SEQ ID NO 30 in the presence of 6 jug/ml lipofectin- The test was consolidated by different ratios of peripheral blood mononuclear cells (PBMCs) to tumor cells (20:1, 10:1, 5:1, 2,5:1, 1,25:1). The respective restoration of the cell mediated cytotoxicity at all of these ratios was 100%. Results were taken from triplicates.

TGF-beta 1: non-small cell lung (NSCLC) carcinoma cell line HCI-H661 Cell mediated cytotoxicity inhibited by high levels of TGF-beta 1, was also was completely restored in the non-small cell lung carcinoma cell line HCI-H661 by 200 nM of SEQ ID NO 30 in the presence of 3 µL/g/ml lipofectin. The test was consolidated by different ratios of peripheral blood mononuclear cells (PBMCs) to tumor cells (20:1, 10:1, 5:1, 2,5:1). The respective restoration of the cell mediated cytotoxicity in all of these ratios was 100%. Results were taken from triplicates.

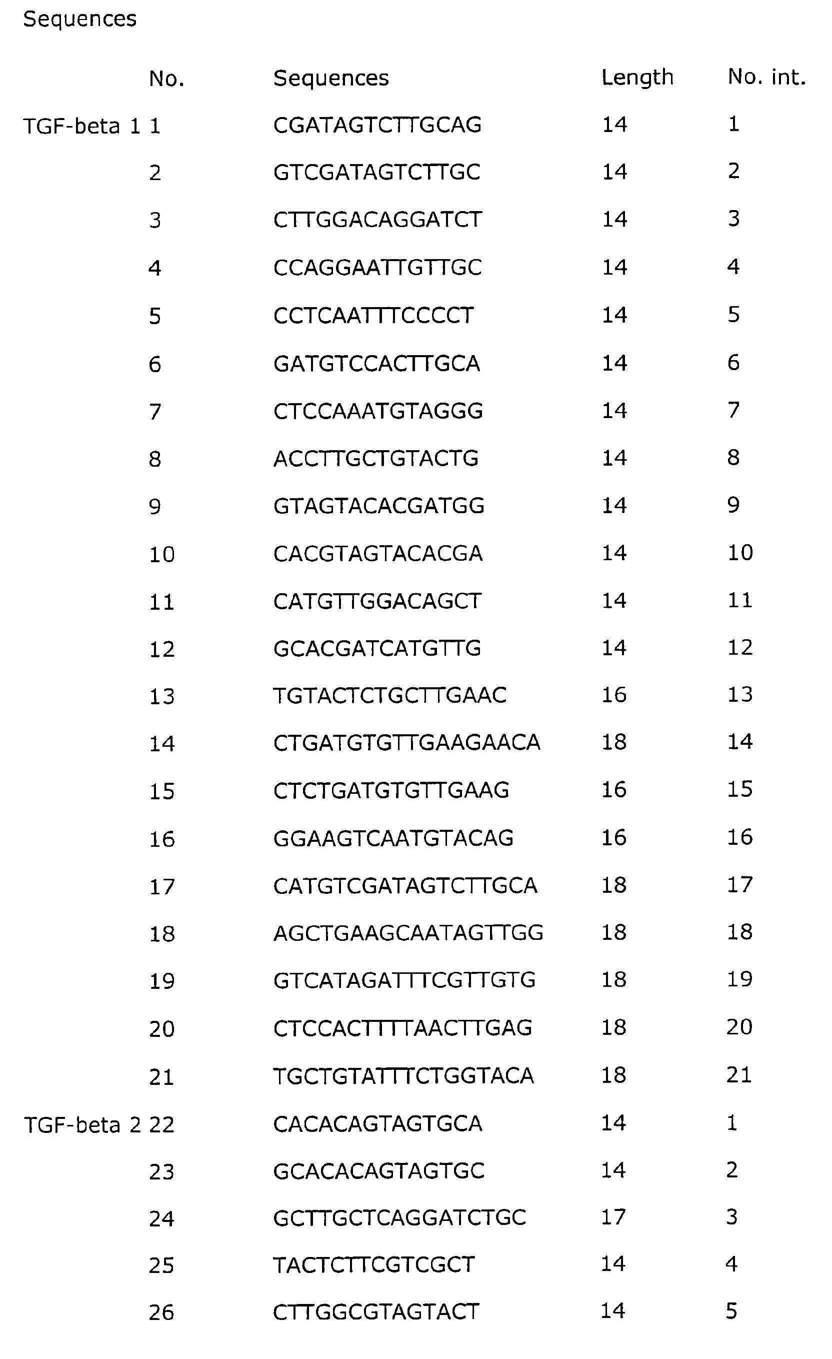

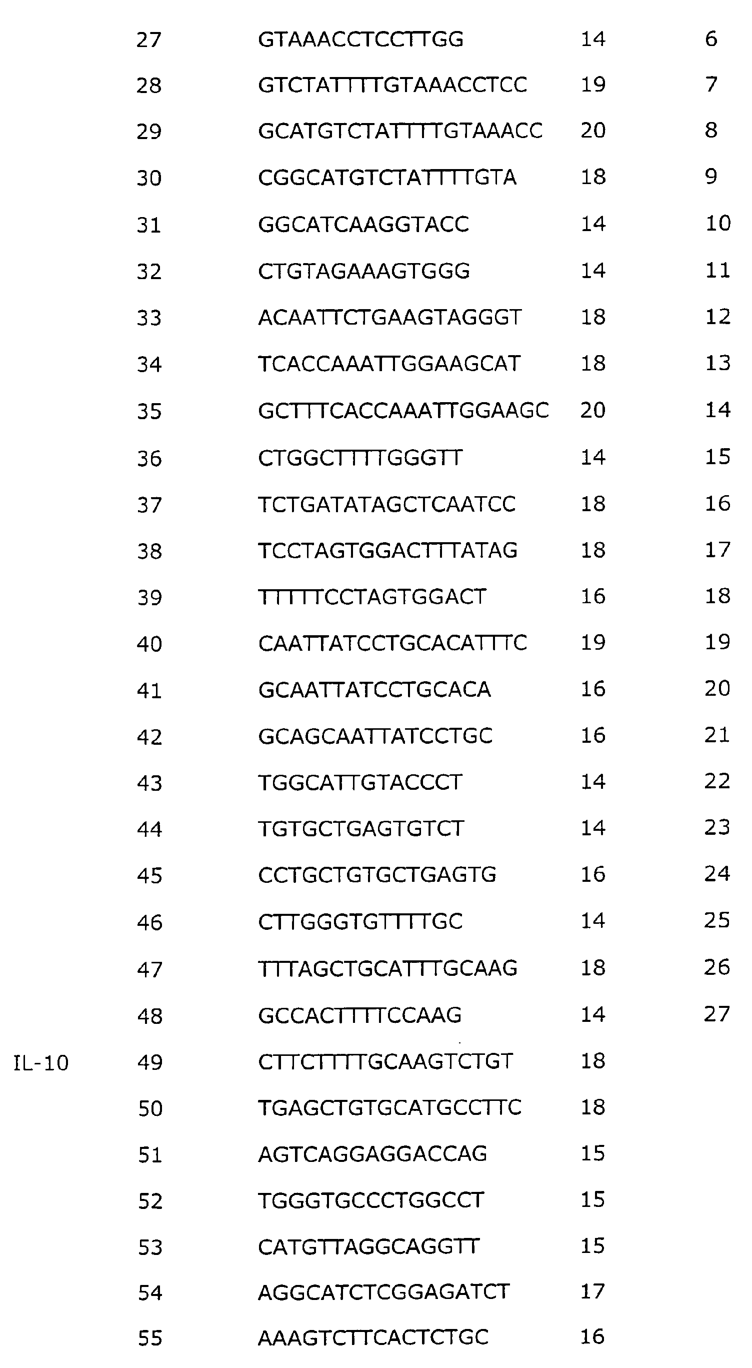

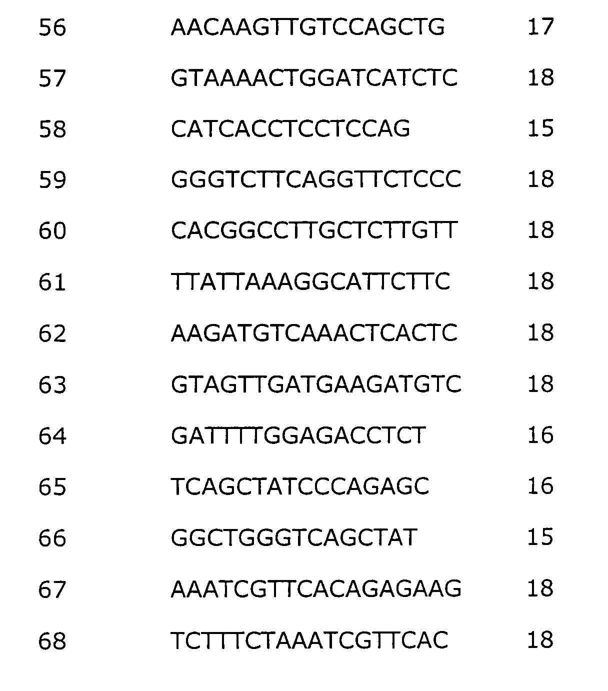

## Claims

1. Use of at least one oligonucleotide or its active derivative for the preparation of a pharmaceutical composition for inhibiting the formation of metastases in cancer treatment.

2. Use according to claim 1 wherein the oligonucleotide is an antisense oligonucleotide inhibiting the synthesis of proteins involved in the formation of metastases.

3. Use according to claim 1 or 2 wherein the oligonucleotide is an antisense oligonucleotide inhibiting the production of tumor growth factor beta 1 (TGF-beta 1), TGF-beta 2, TGF-beta 3, cell-cell adhesion molecules (CAMs), integrins, selectines, metalloproteases (MMPs), their tissue inhibitors (TIMPs) and/or interleukine 10.

4. Use according to any of claims 1 to 3 wherein the oligonucleotide is identified in the sequence listing under SEQ ID NO.1 to 68.

5. Use of oligonucleotides according to any of claims 1 to 4 wherein the oligonucleotide is identified in the sequence listing under SEQ ID NO. 1, 5, 6, 8, 9, 14, 15, 16, 28, 29 30, 34, 35, 36, 40, 42.

6. Use according to any of claim 1 to 5 wherein the cancer is selected from the group of bile duct carcinoma, bladder carcinoma, brain tumor, breast cancer, bronchogenic carcinoma, carcinoma of the kidney, cervical cancer, choriocarcinoma, cystadenocarcinome, cervical carcinoma, colon carcinoma, colorectal carcinoma, embrional carcinoma, endometrial cancer, epithelial carcinoma, esophageal cancer, gallbladder cancer, gastric cancer, head and neck cancer, hepatocellular cancer, liver carcinoma, lung carcinoma, medullary carcinoma, non-small-cell bronchogenic/lung carcinoma, ovarian cancer, pancreas carcinoma, papillary carcinoma, papillary adenocarcinoma, prostate cancer, small intestine carcinoma, rectal cancer, renal cell carcinoma, sebaceous gland carcinoma, skin cancer, small-cell bronchogenic/lung carcinoma, soft tissue cancer, squamous cell carcinoma, testicular carcinoma, uterine cancer, acoustic neuromas, neurofibromas, trachomas, and pyogenic granulomas; pre-malignant tumors, blastoma, Ewing's tumor, craniopharyngloma, ependymoma, medulloblastoma, hemanglioblastoma, medullablastoma, melanoma, mesothelioma, neuroblastoma, neurofibroma, pinealoma, retinoblastoma, retinoblastoma, sarcoma (including angiosarcoma, chondrosarcoma, endothelialsarcoma, fibrosarcoma, gliosarcoma, leiomyosarcoma, liposarcoma, lymphangioandotheliosarcoma, lyphangiosarcoma, melanoma, meningioma, myosarcoma, osteogenic sarcoma, osteosarcoma), seminoma, trachomas, Wilm's tumor.

7. Use according to any of claim 1 to 5 wherein the cancer is selected from the group of prostata cancer, colon carcinoma, endometrial cancer, esophageal cancer, hepatocellular cancer, non-small-cell lung carcinoma, ovarian cancer, pancreas carcinoma and soft tissue cancer.

8. Use of oligonucleotides or their active derivatives for the preparation of a pharmaceutical composition for the treatment of prostate cancer, bladder carcinoma, colon cancer, endometrial cancer, hepatocellular carcinoma, leukemia, lymphoma, melanoma, non-small cell lung cancer (NSCLC), ovarian cancer, pancreatic cancer.

9. Use according to claim 8 wherein the oligonucleotide is an antisense oligonucleotide inhibiting the production of tumor growth factor beta 1 (TGF-beta 1), TGF-beta 3 and/or interleukine 10.

10. Use according to claim 9 wherein the oligonucleotide is identified in the sequence listing under SEQ ID NO. 1 to 21 or 49 to 68.

11. Antisense-oligonucleotide or its active derivative, selected from the group of IL-10 antisense oligonucleotides identified in the sequence listing under Seq. ID. NO 49 to 68.

12. Process of manufacturing the antisense oligonucleotide or its active derivative according to claim 11 by adding consecutive nucleosides and linker stepwise or by cutting the oligonucleotide out of longer oligonucleotide chain.

13. Process according to claim 12 by using phosphite triester chemistry growing the nucleotide chain in 3'-5'direction wherein the respective nucleotide is coupled to the first nucleotide that is covalently attached to the solid phase comprising the steps of
- cleaving 5'DMT protecting group of the previous nucleotide
- adding the respective nucleotide for chain prolongation
- modifying phosphite groups subsequently cap unreacted 5'-hydroxyl groups and cleaving the oligonucleotides from the solid support
- followed by working up the synthesis product

14. Pharmaceutical composition comprising an antisense oligonucleotide according to claim 10.

15. Use of the antisense oligonucleotide according to claim 10 for the preparation of a pharmaceutical composition for the treatment of cancer and/or metastases.
